# EUROPEAN PATENT SPECIFICATION

(11) **EP 2 510 072 B1**
(45) Date of publication and mention of the grant of the patent: **21.12.2016**
(21) Application number: 10776951.5
(22) Date of filing: 11.11.2010
(51) Int. Cl.: C09K 11/06, H05B 33/14, A61N 5/06

(54) **THERAPEUTIC AND COSMETIC ELECTROLUMINESCENT COMPOSITIONS**
THERAPEUTISCHE UND KOSMETISCHE ELEKTROLUMINESZENTE ZUSAMMENSETZUNGEN
COMPOSITIONS ÉLECTROLUMINESCENTES THÉRAPEUTIQUES ET COSMÉTIQUES

(30) Priority: 09.12.2009 EP 09015222
(43) Date of publication of application: 17.10.2012
(73) Proprietor: Merck Patent GmbH, 64293 Darmstadt (DE)
(72) Inventor: PAN, Junyou, 60320 Frankfurt am Main (DE); BUCHHOLZ, Herwig, 60599 Frankfurt am Main (DE); AYDT, Ewald M., 64380 Rossdorf (DE)
(86) International application number: PCT/EP2010/006874
(87) International publication number: WO 2011/069590

(56) References cited:
- EP-A2- 1 782 857
- WO-A1-2010/085180
- US-A1- 2008 096 028
- US-A1- 2008 157 662
- D.A. BERNARDS, S. FLORES-TORRES, H. D. ABRUNA, G. G. MALLIARAS: "Observation of electroluminescence and photovoltaic response in ionic junctions", SCIENCE, vol. 313, 2006, pages 1416-1419, XP002616514,

## Description

The present invention relates inter alia to compositions comprising at least one electroluminescent compound which can be used for the treatment and/or prophylaxis of therapeutic diseases and/or cosmetic conditions. The present invention also relates to devices comprising said compositions and their applications in therapeutic and cosmetic applications.

Phototherapy (also called light therapy) can be employed in a wide range of therapeutic diseases and/or cosmetic (also called aesthetic) conditions. The therapy using light, either from LED or laser, is already being used to treat wounds, injuries, neck pain, osteoarthritis, the side effects of chemotherapy and radiotherapy, for instance.

Often the borders between therapeutic and cosmetic applications are vague and depend on individual circumstances and the assessment of a physician. Often therapeutic conditions are associated with cosmetic consideration. The treatment or prophylaxis of acne, for example, may have both therapeutic and cosmetic components, depending on the degree of the condition. The same accounts for psoriasis, atopic dermatitis and other diseases and/or conditions. Many diseases and conditions are associated with apparent implications which are often represented by a change in the visibility of a subject's skin, for instance. These cosmetic or aesthetic changes can lead to psychological modifications resulting, at least in part, in serious diseases.

Some conditions or diseases may have an emphasis on cosmetic components, even if therapeutic elements may also play a role. Some of these are selected from anti-ageing, anti-wrinkle, the prevention and/or therapy of acne and vitiligo.

Many diagnostic tools or devices may also require light sources, e.g., in order to determine blood characteristics such as bilirubin, oxygen, or CO. In both cosmetics and medicine the skin is the main target to be radiated, but other targets of the human or animal body can also be accessed by phototherapy. These targets include, but are not limited to, the eye, wounds, nails, and internal parts of the body. Light can also be used in order to facilitate or support sterilization and/or disinfection of wounds, beverages, nutrition, for example.

One of the primary effects of phototherapy is the stimulation of metabolism in the mitochondria. Certain wavelengths of light stimulate cytochrome c oxidase, an enzyme which is responsible for the production of the essential cellular energy in the form of adenosine triphosphate (ATP). ATP is required for cellular energy transfer in order to drive thermodynamically unfavoured biochemical reactions and as cellular energy storage. ATP can also act as signal molecule in order to modulate other biochemical molecules (e.g. reactive oxygen species and nitric oxide) that lead to ageing and cell death (oxidative stress). After phototherapy, the cells show an increased metabolism, they communicate better and they survive stressful conditions in a better way.

Such principle can be applied for medicinal therapeutic and cosmetic applications, such as wound healing, connective tissue repair, tissue repair, prevention of tissue death, relief of inflammation, pain, acute injuries, chronic diseases, metabolic disorders, neurogenic pain and seasonal effect disorders.

Another area of the application of light is the treatment of various cancers. In cancer therapy photodynamic therapy (PDT) plays an important role. In PDT light may be used in conjunction with a pharmaceutical compound. These therapies can be used to treat a variety of skin and internal diseases. In PDT, a light-sensitive therapeutic agent known as a photopharmaceutical is supplied externally or internally to an area of the body which is to be treated. That area is then exposed to light of a suitable frequency and intensity to activate the photopharmaceutical. A variety of photopharmaceutical agents are currently available. For example there are topical agents such as 5-aminolevulinic acid hydrochloride (Crawford Pharmaceuticals), methylaminolevulinic acid (Metfix®, Photocure). There are also injectable drugs used primarily for internal malignancies, including Photofin® (from Axcan) and Foscan® (from Biolitech Ltd). Often, the drug is applied in a non-active form that is metabolised to a light-sensitive photopharmaceutical.

In photodynamic therapy, the primary technique for supplying light to the photopharmaceutical is to project light of a suitable wavelength from standalone light sources such as lasers or filtered arc lamps. These sources are cumbersome and expensive, and are therefore only suitable for use in hospitals. This leads to inconvenience for the patient, and high cost for the treatment. High light irradiances are needed in order to treat an acceptable number of patients per day (for the treatment to be cost effective) and to avoid unduly inconveniencing the patient.

WO 98/46130 and US 6096066 disclose arrays of LEDs for use in photodynamic therapy. The small LED sources taught therein result in uneven light incident on the patient. Fabrication of arrays is complicated, because of the large number of connections required. The devices shown therein are designed for hospital treatment.

GB 2360461 discloses a flexible garment which uses a conventional photodynamic therapy light source to produce light which is then transmitted through optical fibres. As such light sources are heavy, the device is not ambulatory and is limited to hospital use.

US 5698866 discloses a light source using over-driven inorganic LEDs. The resulting light output is not even. A heat-sinking mechanism is required, and the device is suitable only for hospital treatment.

WO 93/21842 disclose light sources using inorganic LEDs. Although transportable, the device is not suitable for ambulatory use by a patient at home and clinical treatment is envisaged.

D. A. Bernards et al. (Science, 2006, 313, 1416-1419) disclose electroluminescent and photovoltaic response in ionic junctions.

US 2008/0157662 A1 discloses polymer light emitting electrochemical cells (PLEC) having balanced electron and hole injection.

EP 1 782 857 A2 discloses ambulatory therapeutic light emitting devices.

A further problem with existing approaches is that it can be difficult to achieve uniform illumination with such sources, especially on curved body parts.

An essential prerequisite for the application of light in the fields mentioned above is the device. The commercial available systems nowadays are mostly based on lasers. However, these systems are hospital based, i.e. stationary devices. In order to reduce costs and to increase convenience as well as compliance a portable home-use technology is required. In fact, some research has been devoted in this direction.

Rochester et al. disclosed in GB 24082092 a flexible medical light source comprising flexible light emitting diodes form on flexible substrate and resulting diagnostic devices directed to monitoring blood characteristics (e.g. levels of CO, oxygen, or bilirubin) and photo-therapeutic devices for treatment of ailments.

Vogle Klaus and Kallert Heiko disclosed in EP 018180773 a device for the treatment of skin. The device comprises an potentially flexible organic light emitting diode (OLED) as light source. The device can be integrated in clothes or plaster.

Attili et al. (Br. J. Dermatol. 161(1), 170-173. 2009) published a clinical open pilot study of ambulatory photodynamic therapy (PDT) using a wearable low-irradiance OLEDs in the treatment of nonmelanoma skin cancer, suggesting that OLED-PDT is less painful than conventional PDT with the added advantage of being lightweight, and therefore has the potential for more convenient PDT at home.

Samuel et al. disclosed in EP 1444008B15 an ambulatory device for the use in a therapeutic and/or cosmetic treatment, the device comprises an OLEDs and poly(p-phenylene vinylene) (PPV) used as an example.

EP 1444008 discloses the Devices for the treatment of photodynamic therapy comprising OLEDs.

Organic light emitting diodes have many advantages over their inorganic counterpart (light emitting diodes - LEDs) in that they are intrinsically flexible, and can be coated on large area by, for example, printing technologies, such as ink jet printing and screen printing.

However, in OLEDs active metals, such as Ba and Ca, are used as cathode. Therefore, OLEDs require excellent encapsulation to ensure long lifetime both in storage and in operation. Overall the production of OLEDs, a multilayer structure, is still an elaborate and cost intensive task.

Encapsulation of devices is still a difficult task. Oxygen and humidity can inhibit or destroy the function of OLEDs. There is, therefore, a need for the development of novel thin light sources without the drawbacks as described above.

Furthermore, the establishment of a three dimensional flexible surface is still an unsolved problem, which is a technically complex and cost intensive task. If an OLED is used highly homogeneous layers are required, which is a complicated challenge if the surface of the device is curved.

Surprisingly, organic light emitting electrochemical cells (OLECs) can be used as light sources for the treatment and prophylaxis of medical and/or cosmetic diseases and conditions. OLECs are very simple in their structure and therefore easily prepared. The preparation of devices with curved or three dimensional surfaces is in the case of OLECs less complex as compared to the preparation of such surfaces in OLEDs. This is due to the fact that the requirements relating to homogeneity of the layer is less stringent. Thus, the production costs in particular for mass production are much lower as compared to the ones of OLEDs.

Furthermore, OLECs do not rely on air-sensitive charge-injection layers or metals such as Ba or Cs for electron injection, which further simplifies their preparation and makes them more cost efficient, as compared to OLEDs. This is due to the less stringent requirements for encapsulation of OLECs.

The underlying technology of OLECs differ from the ones of OLEDs or LEDs. Both OLEDs and LEDs are diodes with forward bias and reverse bias. In contrast to OLECs the I-V (current-voltage) curves of both OLEDs and LEDs are asymmetric. They represent semiconductor technologies whereas an OLEC is basically an electrochemical or more precisely an electrolytic cell. Charge transport in OLEDs occurs via the movement of holes and electrons from molecule to molecule until holes and electrons form so called excitons, i.e. electron-hole-pairs. Light is emitted when electrons and holes recombine. In OLECs, upon applying a voltage, the electrolyte is oxidized at the anode and reduced at the cathode.

The molecular cations and anions diffuse under the electrical field and in the meanwhile doping the organic emissive materials until they meet together to form a so called p-n junction. Further an exciton is formed on the organic emissive compounds in the p-n junction. The radiative decay of the exciton leads to the emission of light. The original work and the principle of OLECs has been published by Qibing Pei et al. in Science, 1995, 269, 1086-1088. OLECs show symmetric I-V curves, have low driving voltages, and there is no need for active metals as cathode.

But the time needed for forming p-n junction is long, therefore the turn-on is not instantaneous. Thus, up to date OLECs aren't suitable for display applications. However, therapeutic and cosmetic applications do not require turn-on or response times as display applications.

Another possible type of light emitting device comprising ionic materials is a device with an ionic p-n junction as reported by Daniel A. Bernards, et al., Science 2008, 313, 1416, wherein two layers are laminated together. One of the layers has a mobile anion and the other one has a mobile cation; by ion exchange an ionic p-n junction is formed in the interface between two layers. Here the ionic p-n junction is formed before the voltage is applied. The emission of light can then occur in the p-n junction. A similar light emitting device was also disclosed in US 2007/0157662 A1.

The present invention relates to a device according to claim 1, i.e. the present invention relates to an organic light emitting electrochemical cell (OLEC) comprising at least one ionic species and at least one organic electroluminescent compound for the use in medicine, characterized in that the device comprises a power supply which is a printable battery. The ionic species can be an organic or inorganic ion. Preferably, the ionic species is a mobile ion.
Preferably the composition comprises 3, particularly preferably 2, and very particularly preferably 1 organic electroluminescent compound.

Preferably the composition comprises 3, particularly preferably 2, and very particularly preferably 1 ionic species.

The composition preferably emits light with a specific wavelength or light with a range of wavelengths when a voltage is applied .

Hereby any therapeutic strategy is included, i.e. treatment of a subject with light can be performed with or without a combination with other treatment approaches. Treatment can, for example, carried out with one or more wavelengths in one or more devices according to claim 1. Furthermore, in addition to devices according to claim 1, further light sources using different technologies can be used for the treatment, such as LEDs, OLEDs, and lasers. In addition, the treatment with said devices can be combined with any known treatment strategy using drugs and cosmetics.

If phototherapy is combined with the treatment of chemical compounds such as a drugs and/or cosmetics light can be used to initiate a (photo-) chemical reaction or activation of the chemical compounds, which is called photodynamic therapy (PDT). Phototherapy according to the present invention can also be used in conjunction with chemical compounds without initiating a photochemical reaction or activation. Synergistic effects for the effectiveness and safety of the treatment of a therapeutic disease can arise from sequential, parallel, and overlapping treatment with both light therapy and drugs and/or cosmetics. The drug(s) or cosmetic compound(s), e.g., can be administered first for a specific time period followed by the application of phototherapy using the devices according to claim 1. The time gap between both treatments may also vary, depending on the drug, its photoreactivity, individual circumstances of the subject, and the specific disease or condition. Both treatments may also overlap timely either partly or completely. The exact treatment strategy will depend on the individual circumstances and the severity of the disease or condition.

The combination therapy can have a synergistic effect and can reduce the side effects of traditional treatment strategies (e.g. the side effects of tetracyclines). This is due to the fact, that smaller doses of the drugs may be required when following the combined approach as outlined herein.

Many diagnostic devices comprise light sources for either illumination only or as functional component for the diagnosis itself, e.g. for the determination of blood parameters such as oxygen. The light sources according to claim 1 can also be used for diagnostic purposes. Based on the teaching of the present invention, one skilled in the art will have no problems to develop diagnostic devices for which light sources are required comprising the said compositions.

Treatment is any exposure of a subject to the radiation of the composition. The treatment may be performed by direct contact between the subject and the device comprising the composition or without direct contact between them. The treatment may be outside or inside the subject. Treatment outside the subject may be, for instance, treatment of the skin, wounds, eye, gingival, mucosa, tongue, hair, nail bed, and nails. Treatment inside the subject may be, for instance, blood vessels, heart, breast, lung, or any other organ of the subject. Particular devices are required for most applications inside the subject. One such example may be a stent comprising a device according to the present invention. The said subject may preferably be a human or an animal. The term cosmetic also includes aesthetic applications.

The wavelength of light that is emitted by the composition when incorporated in any kind of electronic device can be precisely tailored by the selection of the appropriate components of the composition, which also includes a defined mixture of compositions and the employment of colour filter and colour converter. Depending on the application of the composition each therapeutic or cosmetic treatment requires a more or less defined wavelength or spectrum of wavelengths to be emitted.

The composition preferably comprises at least one organic electro-luminescent compound which emit light in the range between 200 and 1000 nm, preferably between 300 and 1000 nm, particularly preferably between 300 and 950 nm, and very particularly preferably between 400 and 900 nm.

As outlined above one of the primary effects of phototherapy is the stimulation of metabolism in the mitochondria. After phototherapy, the cells show an increased metabolism, they communicate better and they survive stressful conditions in a better way.

The devices according to the present invention can be used for cellular stimulation. Preferred wavelengths or ranges of wavelengths for cellular stimulation are in the range between 600 to 900 nm, particularly preferable between 620 and 880 nm, and very particularly preferably between 650 and 870 nm. Examples of particularly preferred wavelengths for cellular stimulation are 683.7, 667.5, 772.3, 750.7, 846, and 812.5 nm.

Any therapeutic disease and/or cosmetic condition approachable by phototherapy can be treated with the OLECs according to claim 1. These diseases and/or conditions include, e.g., skin diseases, and skin-related conditions including skin-ageing, and cellulite, enlarged pores, oily skin, folliculitis, precancerous solar keratosis, skin lesion, aging, wrinkled and sun-damaged skin, crow's feet, skin ulcers (diabetic, pressure, venous stasis), acne rosacea lesions, cellulite; photomodulation of sebaceous oil glands and the surrounding tissues; reducing wrinkles, acne scars and acne bacteria, inflammation, pain, wounds, psychological and neurological related diseases and conditions, edema, Pagets disease, primary and metastatic tumors, connective tissue disease, manipulation of collagen, fibroblast, and fibroblast derived cell levels in mammalian tissue, illuminating retina, neoplastic, neovascular and hypertrophic diseases, inflammation and allergic reactions, perspiration, sweating and hyper-hydrosis from eccrine (sweat) or apocrine glands, jaundice, vitiligo, ocular neovascular diseases, bulimia nervosa, herpes, seasonal affective disorders, mood, sleep disorders, skin cancer, crigler naijar, atopic dermatitis, diabetic skin ulcers, pressure ulcers, bladder infections, relief of muscular pains, pain, stiffness of joints, reduction of bacteria, gingivitis, whitening teeth, treatment of teeth and tissue in mouth, wound healing.

Cosmetic conditions are selected from acne, skin rejuvenation and skin wrinkles, cellulite, and vitiligo. Many therapeutic treatments also have cosmetic component. Psoriasis, e.g., can be mild, mild-to-moderate, moderate, moderate-to-severe and severe. Any of these categories has a cosmetic component, which may be responsible for severe psychological problems of affected patients.

Preferably the said devices are used for the treatment and/or prophylaxis of humans and/or animals. Preferably the device according to the present invention is used for the treatment and/or prophylaxis of humans.

Further subjects suitable to be treated by the irradiation with devices according to the present invention are plants, microbes, bacteria, fungi, and liquids. Microbes include, but are not limited to, prokaryotes such as bacteria and archaea and eukaryotes such as protists, animals, fungi and plants. Preferred liquids are beverages and particularly preferably water.

The said organic electroluminescent compound can be selected from small molecules, polymers, oligomers, dendrimers, blends or mixtures thereof.

The ionic species can be selected from small molecules, polymers, oligomers, dendrimers, blends or mixtures thereof.

The term small molecule as used herein is defined as molecule not being a polymer, oligomer, dendrimer, or a blend. In particular, repeating structures are absent in small molecules. The molecular weight of small molecules is typically in the range of polymers with a low number of repeating units, oligomers or less.

The molecular weight of the small molecule is preferably below 4000 g/mol, particularly preferably below 3000 g/mol, and very particularly preferably below 2000 g/mol.

The polymers of the present invention preferably have 10 to 10000, particularly preferably 20 to 5000 and very particularly preferably 50 to 2000 repeat units. Oligomers according to this invention have preferably 2 to 9 repeat units. The branching index of the polymers and oligomers is between 0 (linear polymer without branching) and 1 (completely branched dendrimer). The term dendrimer as used herein is defined according to M. Fischer et al. in Angew. Chem., Int. Ed. 1999, 38, 885).

The molecular weight (MW) of the polymers of the present invention is preferably in the range of 10000 to 2000000 g/mol, particularly preferably in the range of 100000 to 1500000 g/mol, and very particularly preferably in the range of 200000 to 1000000 g/mol. The determination of MW can be performed according to standard techniques known to the person skilled in the art by employing gel permeation chromatography (GPC) with polystyrene as internal standard, for instance.

A blend is a mixture comprising at least one polymeric dendrimeric, or oligomeric component.

The compositions may also comprise further compounds.

One of the preferred further compounds is selected from hole transport materials (HTM). A HTM is characterized in that it is a material or unit capable of transporting holes (i.e. positive charges).

In principle any HTM known to one skilled in the art can be employed in the compositions. A HTM is preferably selected from amines, triarylamine, thiophenes, carbazoles, phthalocyanines, porphyrines, isomers and derivatives thereof. HTM is particularly preferably selected, from amines, triarylamines, thiophenes, carbazoles, phthalocyanines, and porphyrines.

Suitable materials are phenylenediamine derivatives (US 3615404), arylamine derivatives (US 3567450), amino-substituted chalcone derivatives (US 3526501), styrylanthracene derivatives (JP A 56-46234), polycyclic aromatic compounds (EP 1009041), polyarylalkane derivatives (US 3615402), fluorenone derivatives (JP A 54-110837), hydrazone derivatives (US 3717462), stilbene derivatives (JP A 61-210363), silazane derivatives (US 4950950), polysilanes (JP A 2-204996), aniline copolymers (JP A 2-282263), thiophene oligomers, polythiophenes, PVK, polypyrroles, polyanilines and further copolymers, porphyrin compounds (JP A 63-2956965), aromatic dimethylidene-type compounds, carbazole compounds, such as, for example, CDBP, CBP, mCP, aromatic tertiary amine and styrylamine compounds (US 4127412), and monomeric triarylamine (US 3180730). Even more triarylamino groups may also be present in the molecule.

Preference is given to aromatic tertiary amines containing at least two tertiary amine units (US 4720432 and US 5061569), such as, for example, 4,4'-bis[N-(1-naphthyl)-N-phenylamino]biphenyl (NPD) (US 5061569) or MTDATA (JP A 4-308688), N,N,N',N'-tetra(4-biphenyl)diaminobiphenylene (TBDB), 1,1-bis(4-di-p-tolylaminophenyl)cyclohexane (TAPC), 1,1-bis(4-dip-tolylaminophenyl)-3-phenylpropane (TAPPP), 1,4-bis[2-[4-[N,N-di(p-tolyl)amino]phenyl]vinyl]benzene (BDTAPVB), N,N,N',N'-tetra-p-tolyl-4,4'-diaminobiphenyl (TTB), TPD, N,N,N',N'-tetraphenyl-4,4"'-diamino-1,1':4',1":4",1"'-quaterphenyl, likewise tertiary amines containing carbazole units, such as, for example, 4 (9H-carbazol-9-yl)-N,N-bis[4-(9H-carbazol-9-yl)phenyl]benzeneamine (TCTA). Preference is likewise given to hexaazatriphenylene compounds in accordance with US 2007/0092755 A1.

Particular preference is given to the following triarylamine compounds of the Formulae (1) to (6), which may also be substituted, and as disclosed in EP 1162193 A1, EP 650955 A1, Synth. Metals 1997, 91(1-3), 209, DE 19646119 A1, WO 2006/122630 A1, EP 1860097 A1, EP 1834945 A1, JP 08053397 A, US 6251531 B1, and WO 2009/041635.

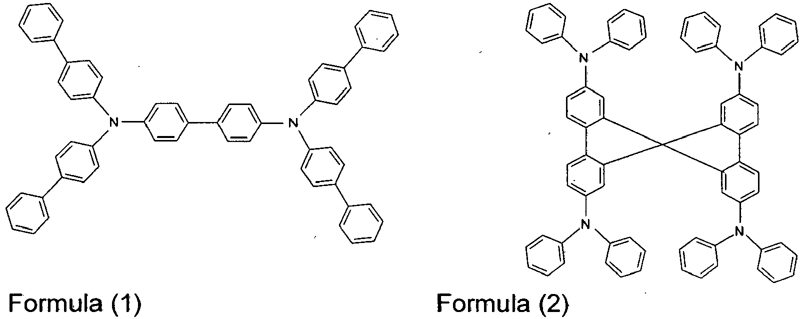

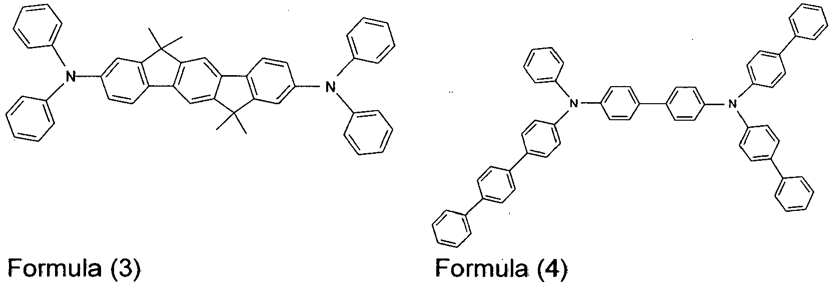

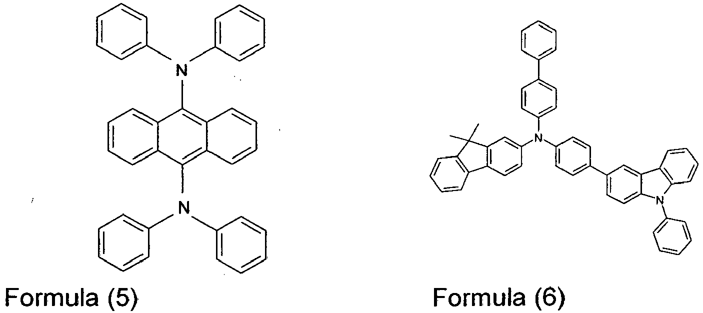

One of the preferred further compounds is selected from electron transport materials (ETM). An ETM refers to a material capable of transporting electrons (i.e. negative charges). In principle any ETM known to one skilled in the art can be employed in the compositions. Suitable ETMs are selected from the group consisting of imidazoles, pyridines, pyrimidines, pyridazines, pyrazines, oxadiazoles, chinolines, chinoxalines, anthracenes, benzanthracenes, pyrenes, perylenes, benzimidazoles, triazines, ketones, phosphinoxides, phenazines, phenanthrolines, triarylboranes, isomers and derivatives thereof.

Suitable ETMs are metal chelates of 8 hydroxyquinoline (for example Liq, Alq₃, Gaq₃, Mgq₂, Znq₂, Inq₃, Zrq₄), Balq, 4 azaphenanthrene-5-ol/Be complexes (US 5529853 A; e.g. Formula (7)), butadiene derivatives (US 4356429), heterocyclic optical brighteners (US 4539507), benzazoles, such as, for example, 1,3,5-tris(2-N-phenylbenzimidazolyl)benzene (TPBI) (US 5766779, Formula (8)), 1,3,5-triazines, pyrenes, anthracenes, tetracenes, fluorenes, spirobifluorenes, dendrimers, tetracenes, for example rubrene derivatives, 1,10-phenanthroline derivatives (JP 2003/115387, JP 2004/311184, JP 2001/267080, WO 2002/043449), silacyl-cyclopentadiene derivatives (EP 1480280, EP 1478032, EP 1469533), pyridine derivatives (JP 2004/200162 Kodak), phenanthrolines, for example BCP and Bphen, also a number of phenanthrolines bonded via biphenyl or other aromatic groups (US 2007/0252517 A1) or phenanthrolines bonded to anthracene (US 2007/0122656 A1, e.g. Formulae (9) and (10)), 1,3,4-oxadiazoles, for example Formula (11), triazoles, for example Formula (12), triarylboranes, benzimidazole derivatives and other N heterocyclic compounds (cf. US 2007/0273272 A1), silacyclopentadiene derivatives, borane derivatives, Ga oxinoid complexes.

Preference is given to 2,9,10-substituted anthracenes (with 1- or 2-naphthyl and 4- or 3-biphenyl) or molecules which contain two anthracene units (US 2008/0193796 A1).

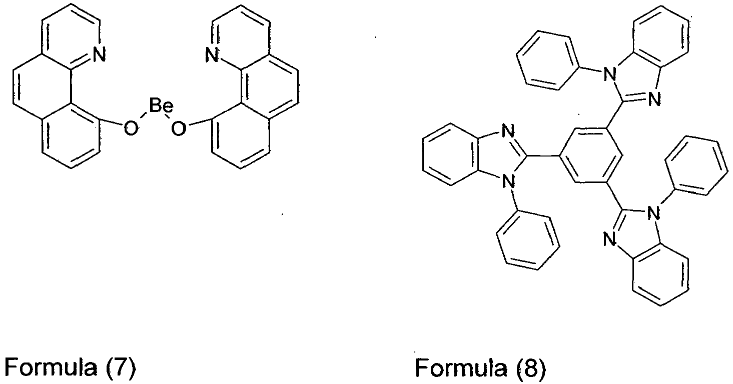

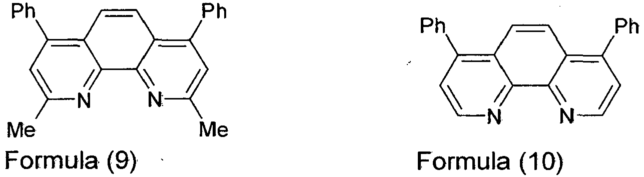

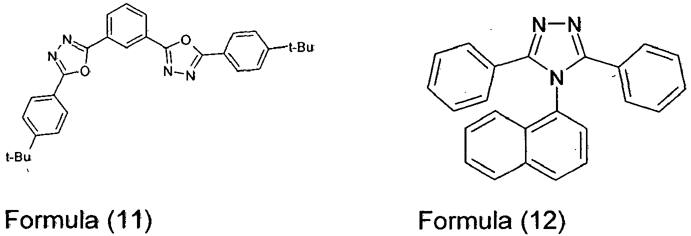

Preference is likewise given to anthracene-benzimidazole derivatives, such as, for example, the compounds of Formulae (13) to (15), and as disclosed in, e.g., US 6878469 B2, US 2006/147747 A, and EP 1551206 A1.

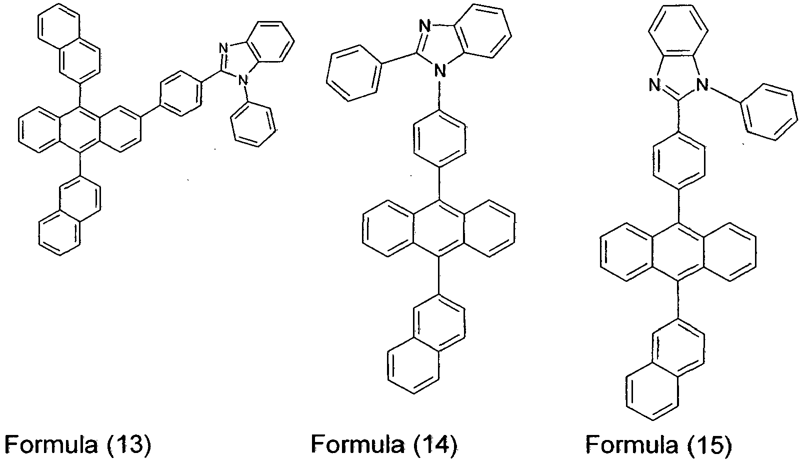

In a preferred embodiment, the said composition further comprises an ionic conductor, which is preferably selected from polymeric materials, such as perfluorosulfonic acid-based formulations, polybenzimidazoles, sulfonated polyetherketone, sulfonated naphthalenic polyimides, and polyethylene oxide (PEO)-based formulations. Further suitable polymers can be selected from the polymers for proton-exchange membrane for fuel cells. Such polymers are disclosed, e.g., in the review of Hickner et al., "Alternative Polymer Systems for Proton Exchange Membranes (PEMs)" in Chemical Reviews, 2004, 104, 4587-4612. A very preferred ion conductor for the present invention is polyethylene oxide (PEO).

The composition comprises 0 to 50 wt%, preferably 10 to 40 wt%, particularly preferably 10 to 30 wt%, and very particularly preferably 15 to 25 wt% of the HTM with respect to the composition.

The composition comprises 0 to 50 wt%, preferably 10 to 40 wt%, particularly preferably 10 to 30 wt%, and very particularly preferably 15 to 25 wt% of the ETM with respect to the composition.

In the case the composition comprises one organic electroluminescent compound and separate ionic materials, the composition comprises 0.1 to 20 wt%, preferably 1 to 15 wt%, particularly preferably 2 to 10 wt%, and very particularly preferably 5 to 10 wt% of the ionic species with respect to the composition and 20 to 99.9 wt%, preferably 20 to 80 wt%, particularly preferably 20 to 70 wt%, and very particularly preferably 20 to 50 wt% of the organic electroluminescent compound with respect to the composition.

Optionally the composition further comprises at least one ion conductor compound which can have a concentration of 0 to 60 wt%, preferably 10 to 60 wt%, particularly preferably 20 to 50 wt%, and very particularly preferably 30 to 50 wt% with respect to the composition.

The composition can comprise at least one ionic organic electroluminescent compound in form of K⁺A⁻, wherein either K⁺ or A⁻ is an organic emissive material. Preferably the composition comprises 3, particularly preferably 2, and very particularly preferably 1 compound of the formula K⁺A⁻.

One typical material class is the so-called ionic transition metal complexes (iTMCs) as reported for example by Rudmann et al., J. Am. Chem. Soc. 2002, 124, 4918-4921 and Rothe et al., Adv. Func. Mater. 2009, 19, 2038-2044. The composition preferably comprises further ion conducting material or a neutral matrix material, which can have a concentration of 0 to 70wt%, preferably 10 to 60 wt%, particularly preferably 10 to 40 wt%, and very particularly preferably 20 to 30 wt% with respect to the composition.

Preference is given to a composition, characterized in that the at least one of the organic electroluminescent compounds is selected from fluorescent emitter materials, phosphorescent emitter materials, and emissive organo metallic complexes.

The term electroluminescent compound refers to a material which, upon receiving energy by applying a voltage, undergoes radiative decay to emit light.

There are two classes of emitters or emitter materials, fluorescent and phosphorescent emitters. The term fluorescent emitter relates to materials or compounds which undergo a radiative transition from an excited singlet state to its ground. The term phosphorescent emitter, as used herein, relates to luminescent materials or compounds which comprise transition metals. This typically includes materials emitting light caused by spin forbidden transition(s), e.g., transitions from excited triplet and/or quintet states.

According to quantum mechanics the transition from excited states with high spin multiplicity, e.g. from excited triplet states, to ground state is forbidden. However, the existence of an heavy atom, for example iridium, osmium, platinum and europium, results in a strong spin-orbit coupling, i.e. the excited singlet and triplet are mixed so that triplet gains some singlet character; and if singlet -triplet mixing yields a radiative decay rate faster than the non-radiative event, then the luminance can be efficient. This kind of emission can be achieved using metal complex, as reported by Baldo et al.; Nature 395, 151-154 (1998).

The term dopant as employed herein is also used for the term emitter, emitter material, or emissive material.

Particular preference is given to organic electroluminescent compounds selected from fluorescent emitter.

Emitter compounds tend to have an extended conjugated π-electron systems. Many examples have been published, e.g. styrylamine derivatives as disclosed in JP 2913116B and WO 2001/021729 A1, and indenofluorene derivatives as disclosed in WO 2008/006449 and WO 2007/140847.

Blue fluorescent emitters are preferably polyaromatic compounds, such as, for example, 9,10-di(2-naphthylanthracene) and other anthracene derivatives, derivatives of tetracene, xanthene, perylene, such as, for example, 2,5,8,11-tetra-t-butylperylene, phenylene, for example 4,4'-(bis(9-ethyl-3-carbazovinylene)-1,1'-biphenyl, fluorene, arylpyrenes (US 2006/0222886), arylenevinylenes (US 5121029, US 5130603), derivatives of rubrene, coumarine, rhodamine, quinacridone, such as, for example, N,N'-dimethylquinacridone (DMQA), dicyanomethylenepyrane, such as, for example, 4 (dicyanoethylene)-6-(4-dimethylaminostyryl-2-methyl)-4H-pyrane (DCM), thiopyrans, polymethine, pyrylium and thiapyrylium salts, periflanthene, indenoperylene, bis(azinyl)imine-boron compounds (US 2007/0092753 A1), bis(azinyl)methene compounds and carbostyryl compounds.

Further preferred blue fluorescent emitters are described in C.H. Chen et al.: "Recent developments in organic electroluminescent materials" Macromol. Symp. 125, (1997), 1-48 and "Recent progress of molecular organic electroluminescent materials and devices" Mat. Sci. and Eng. R, 39 (2002), 143-222.

Preferred fluorescent dopants are selected from the class of the monostyrylamines, the distyrylamines, the tristyrylamines, the tetrastyrylamines, the styrylphosphines, the styryl ethers and the arylamines.

A monostyrylamine is taken to mean a compound which contains one substituted or unsubstituted styryl group and at least one, preferably aromatic, amine. A distyrylamine is taken to mean a compound which contains two substituted or unsubstituted styryl groups and at least one, preferably aromatic, amine. A tristyrylamine is taken to mean a compound which contains three substituted or unsubstituted styryl groups and at least one, preferably aromatic, amine. A tetrastyrylamine is taken to mean a compound which contains four substituted or unsubstituted styryl groups and at least one, preferably aromatic, amine. The styryl groups are particularly preferably stilbenes, which may also be further substituted. The corresponding phosphines and ethers are defined analogously to the amines. For the purposes of this invention, an arylamine or an aromatic amine is taken to mean a compound which contains three substituted or unsubstituted aromatic or heteroaromatic ring systems bonded directly to the nitrogen. At least one of these aromatic or heteroaromatic ring systems is preferably a condensed ring system, preferably having at least 14 aromatic ring atoms. Preferred examples thereof are aromatic anthracene-amines, aromatic anthracene-diamines, aromatic pyrene-amines, aromatic pyrene-diamines, aromatic chrysene-amines and aromatic chrysene-diamines. An aromatic anthracene-amine is taken to mean a compound in which one diarylamino group is bonded directly to an anthracene group, preferably in the 9 position. An aromatic anthracene-diamine is taken to mean a compound in which two diarylamino groups are bonded directly to an anthracene group, preferably in the 9,10-position. Aromatic pyrene-amines, pyrene-diamines, chrysene-amines and chrysene-diamines are defined analogously thereto, where the diarylamino groups on the pyrene are preferably bonded in the 1 position or in the 1,6-position.

Further preferred fluorescent dopants are selected from indenofluoreneamines and indenofluorene-diamines, for example in accordance with WO 2006/122630, benzoindenofluorene-amines and benzoindenofluorene-diamines, for example in accordance with WO 2008/006449, and dibenzoindenofluorene-amines and dibenzoindenofluorene-diamines, for example in accordance with WO 2007/140847.

Examples of dopants from the class of the styrylamines are substituted or unsubstituted tristilbene-amines or the dopants described in
WO 2006/000388, WO 2006/058737, WO 2006/000389,
WO 2007/065549 and WO 2007/115610. Distyrylbenzene and distyrylbiphenyl derivatives are described in US 5121029. Further styrylamines are found in US 2007/0122656 A1.

Particularly preferred styrylamine dopants and triarylamine dopants are the compounds of the Formulae (16) to (21) and as disclosed in
US 7250532 B2, DE 102005058557 A1, CN 1583691 A, JP 08053397 A,
US 6251531 B1, and US 2006/210830 A.

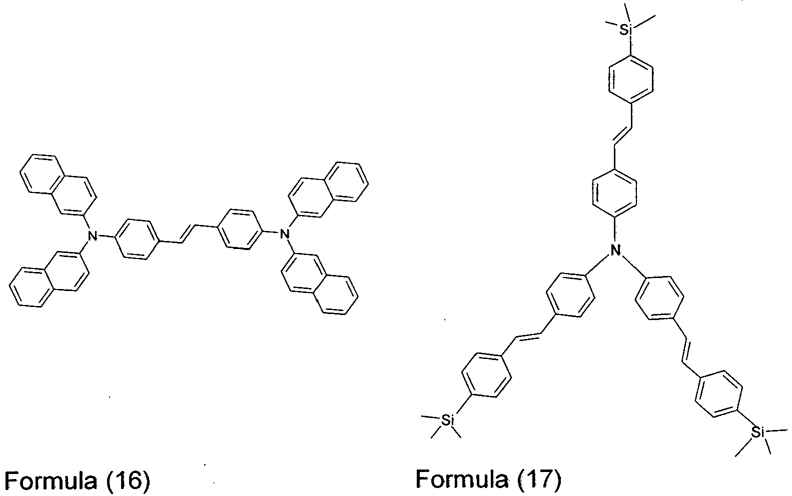

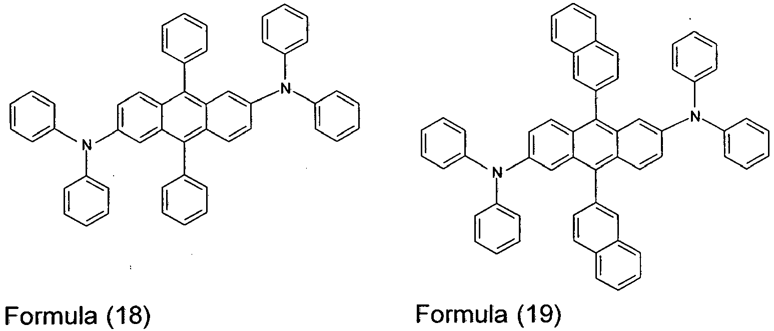

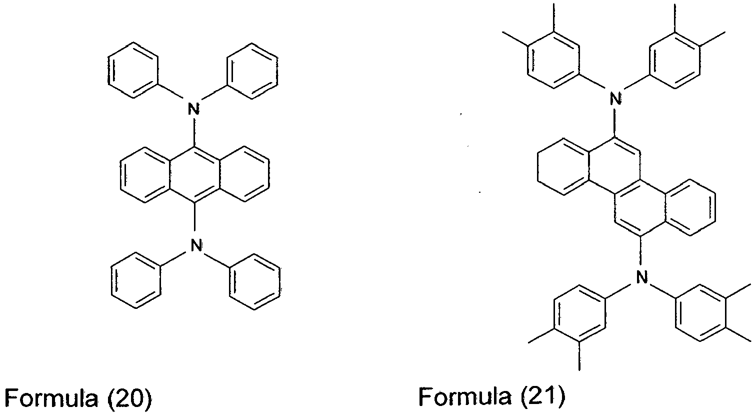

Further preferred fluorescent dopants are selected from the group of triarylamines as disclosed in EP 1957606 A1and US 2008/0113101 A1.

Further preferred fluorescent dopants are selected from derivatives of naphthalene, anthracene, tetracene, fluorene, periflanthene, indenoperylene, phenanthrene, perylene (US 2007/0252517 A1), pyrene, chrysene, decacyclene, coronene, tetraphenylcyclopentadiene, pentaphenylcyclopentadiene, fluorene, spirofluorene, rubrene, coumarine (US 4769292, US 6020078, US 2007/0252517 A1), pyran, oxazone, benzoxazole, benzothiazole, benzimidazole, pyrazine, cinnamic acid esters, diketopyrrolopyrrole, acridone and quinacridone (US 2007/0252517 A1).

Of the anthracene compounds, particular preference is given to 9,10-substituted anthracenes, such as, for example, 9,10-diphenylanthracene and 9,10-bis(phenylethynyl)anthracene. 1,4-Bis(9'-ethynylanthracenyl)benzene is also a preferred dopant.

Particular preference is given to organic electroluminescent compounds selected from phosphorescent emitter.

Examples of phosphorescent emitters are disclosed in the applications WO 00/70655, WO 01/41512, WO 02/02714, WO 02/15645, EP 1191613, EP 1191612, EP 1191614 and WO 2005/033244. In general, all phosphorescent complexes as used in accordance with the prior art and as are known to the person skilled in the art in the area of organic electroluminescence are suitable, and the person skilled in the art will be able to use further phosphorescent complexes without inventive step.

The phosphorescent emitter may be a metal complex, preferably with the formula M(L)_{z}, wherein M is a metal atom, L is in each occurrence independently of one another an organic ligand that is bonded to or coordinated with M via one, two or more positions, and z is an integer ≥ 1, preferably 1, 2, 3, 4, 5 or 6, and wherein, optionally, these groups are linked to a polymer via one or more, preferably one, two or three positions, preferably via the ligands L.

M is in particular a metal atom selected from transition metals, preferably selected from transition metals of group VIII, or lanthanoides, or actinides, particularly preferably selected from Rh, Os, Ir, Pt, Pd, Au, Sm, Eu, Gd, Tb, Dy, Re, Cu, Zn, W, Mo, Pd, Ag, or Ru, and very particularly preferably selected from Os, Ir, Ru, Rh, Re, Pd, or Pt. M may also be Zn.

Preferred ligands are 2 phenylpyridine derivatives, 7,8-benzoquinoline derivatives, 2 (2-thienyl)pyridine derivatives, 2 (1-naphthyl)pyridine derivatives or 2 phenylquinoline derivatives. All these compounds may be substituted, for example by fluoro- or trifluoromethyl substituents for blue. Auxiliary ligands are preferably acetylacetonate or picric acid.

In particular, complexes of Pt or Pd with tetradentate ligands of the Formula (22) as disclosed in US 2007/0087219 A1, wherein R¹ to R¹⁴ and Z¹ to Z⁵ are as defined in the reference, Pt porphyrin complexes having an enlarged ring system (US 2009/0061681 A1) and Ir complexes are suitable, for example 2,3,7,8,12,13,17,18-octaethyl-21H, 23H-porphyrin-Pt(II), tetraphenyl-Pt(II)-tetrabenzoporphyrin (US 2009/0061681 A1), cis-bis(2-phenylpyridinato-N,C2')Pt(II), cis-bis(2-(2'-thienyl)pyridinato-N,C3')Pt(II), cis-bis(2-(2'-thienyl)quinolinato-N,C5')Pt(II), (2-(4,6-difluorophenyl)pyridinato-N,C2')Pt(II) acetylacetonate, or tris(2-phenylpyridinato-N,C2')Ir(III) (Ir(ppy)₃, green), bis(2-phenylpyridinato-N,C2)Ir(III) acetylacetonate (Ir(ppy)₂ acetylacetonate, green,
US 2001/0053462 A1, Baldo, Thompson et al. Nature 403, (2000), 750-753), bis(1-phenylisoquinolinato-N,C2')(2-phenylpyridinato-N,C2')iridium(III), bis(2-phenylpyridinato-N,C2')(1-phenylisoquinolinato-N,C2')iridium(III), bis(2-(2'-benzothienyl)pyridinato-N,C3')iridium(III) acetylacetonate, bis(2-(4',6'-difluorophenyl)pyridinato-N,C2')iridium(III) piccolinate (Firpic, blue), bis(2-(4',6'-difluorophenyl)pyridinato-N,C2')Ir(III) tetrakis(1-pyrazolyl)borate, tris(2-(biphenyl-3-yl)-4-tert-butylpyridine)iridium(III), (ppz)₂Ir(5phdpym) (US 2009/0061681 A1), (45ooppz)₂Ir(5phdpym) (US 2009/0061681 A1), derivatives of 2 phenylpyridine-Ir complexes, such as, for example, iridium(III) bis(2-phenylquinolyl-N,C2')acetylacetonate (PQIr), tris(2-phenylisoquinolinato-N,C)Ir(III) (red), bis(2-(2'-benzo[4,5-a]thienyl)pyridinato-N,C3)Ir acetylacetonate ([Btp2Ir(acac)], red, Adachi et al. Appl. Phys. Lett. 78 (2001), 1622-1624).

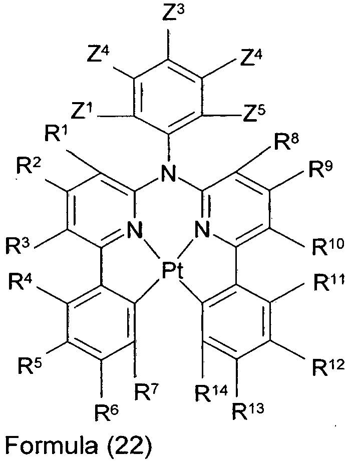

Also suitable are complexes of trivalent lanthanides, such as, for example, Tb³⁺ and Eu³⁺ (J. Kido et al. Appl. Phys. Lett. 65 (1994), 2124, Kido et al. Chem. Lett. 657, 1990, US 2007/0252517 A1), or phosphorescent complexes of Pt(II), Ir(I), Rh(I) with maleonitrile dithiolate (Johnson et al., JACS 105, 1983, 1795), Re(I) tricarbonyl diimine complexes (Wrighton, JACS 96, 1974, 998 inter alia), Os(II) complexes with cyano ligands and bipyridyl or phenanthroline ligands (Ma et al., Synth. Metals 94, 1998, 245) or Alq₃.

Further phosphorescent emitters with tridentate ligands are described in US 6824895 and US 7029766. Red-emitting phosphorescent complexes are mentioned in US 6835469 and US 6830828.

A particularly preferred phosphorescent dopant is a compound with the Formula (23) and further compounds as disclosed, e.g., in
US 200170053462 A1.

A particularly preferred phosphorescent dopant is a compound with the Formula (24) and further compounds as disclosed, e.g., in
WO 2007/095118 A1

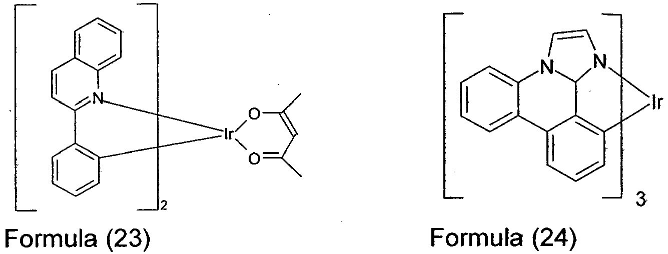

Further derivatives are described in US 7378162 B2, US 6835469 B2, and JP 2003/253145 A.

Particular preference is given to organic electroluminescent compounds selected from organo metallic complexes.

Further to metal complexes mentioned elsewhere herein, a suitable metal complex according to the present invention can be selected from transition metals, rare earth elements, lanthanides and actinides is also subject of this invention. Preferably the metal is selected from Ir, Ru, Os, Eu, Au, Pt, Cu, Zn, Mo, W, Rh, Pd, or Ag.

In a preferred embodiment, the organic electroluminescent compound emits in ultraviolet (UV) range. Suitable UV emitter materials can be selected from organic compounds comprising a wide-gap between the highest occupied molecular orbital (HOMO) and the lowest unoccupied molecular orbital (LUMO) moieties with a small π-conjugated system. Such UV emitter can be preferably selected from small molecular compounds comprising carbazoles, indenocarbazole, indolocarbazole, silane, fluorene, triazine, thiophene, dibenzothiophene, furane, dibenzofurane, imidazole, benzimidazole, anthracene, naphthalene, phenanthrene, amine, triarylamine and derivatives theirof.

In another preferred embodiment, the suitable UV emitter can be selected from polymeric materials which have a limited conjugated length, for example a spiro-bifluorene polymer as reported by Wong, Ken Tsung et al. (Org. Lett. 2005, 7, 5131) and fluorene polymers as reported by Chao, Teng Chih, et al. (Adv. Mater. (Weinheim, Ger.) 2005, 17, 992).

Preferably, the polymeric UV emitter materials are selected from non-conjugated polymers, which comprises the small molecular UV emitter as described above. The suitable non-conjugated polymer can be a side-chain polymer with emitter and other functional groups on the side-chains, as, for example, disclosed in JP 2005/108556, JP 2005/285661,
JP 2003/338375, or a non-conjugated main chain polymer, as disclosed for example in US 7279702 B2, DE 102009023154.4, and DE 102009023156.0

The organic electroluminescent compound may also be a polymer, oligomer, dendrimer, and blend.

The polymer may also have further functions such as charge transfer transport function. Therefore, the compositions can also comprise further polymeric molecules.

Preferably, the said polymer comprises units, which are preferably selected from the groups comprising phosphorescent emitter, particularly emissive metal complexes as described above. Particular preference is given here to corresponding structural units which contain elements from groups 8 to 10 (Ru, Os, Rh, Ir, Pd, Pt).

The polymer is characterized in that different functions may be incorporated into one large molecule or a blend of large molecules. The functions are, inter alia, the ones of a hole injection material, hole transport material, emissive material, electron injection material, and electron transport material. The functions which are incorporated into a polymer can be categorized into different groups. By choosing the desired functional groups and the ratio between them, the polymer can be tuned to have the desired function(s).

The difference between polymers, oligomers and dendrimers is due to the size, size distribution, and branching of the molecular entities as defined above.

Different structures are, inter alia, those as disclosed and extensively listed in WO 2002/077060 A1 and in DE 10337346 A1. The structural units may originate, for example, from the following groups:
- Group 1:: units which increase the hole-injection and/or transport properties of the polymers; It corresponds to the HIMs or HTMs as described above.
- Group 2:: units which increase the electron-injection and/or transport properties of the polymers; It corresponds to the ElMs or ETMs as described above.
- Group 3:: units which have combinations of individual units from group 1 and group 2;
- Group 4:: units which modify the emission characteristics to such an extent that electrophosphorescence may be obtained instead of electrofluorescence; typically, it corresponds to the phosphorescent emitter, or more preferably emissive metal complexes as described above.
- Group 5:: units which improve the transition from the so called singlet state to higher spin states, e.g. to a triplet state;
- Group 6:: units which influence the morphology and/or emission colour of the resultant polymers;
- Group 7:: units which are typically used as backbone and which may have electron transport function, hole transport function or both.

Preferably, the polymer is a hole transport or injection polymer comprising units of groups 1, which are preferably selected from units comprising the low molecular weight HTMs or HIMs as described above.

Further preferred units from group 1 are, for example, triarylamine, benzidine, tetraaryl-para-phenylenediamine, carbazole, azulene, thiophene, pyrrole and furan derivatives and further O, S, or N containing heterocycles.

Preferred polymeric HTM or HIM is a polymer comprising at least one of following repeat unit according to Formulae (25). 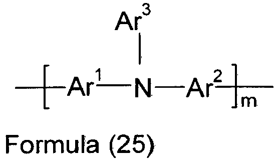 wherein
Ar¹ which may be the same or different, denote, independently if in different repeat units, a single bond or an optionally substituted mononuclear or polynuclear aryl group,
Ar² which may be the same or different, denote, independently if in different repeat units, an optionally substituted mononuclear or polynuclear aryl group,
Ar³ which may be the same or different, denote, independently if in different repeat units, an optionally substituted mononuclear or polynuclear aryl group,
m is 1, 2 or 3.

Particularly preferred units of Formula (25) are selected from the group consisting of the Formulae (26) to (28): 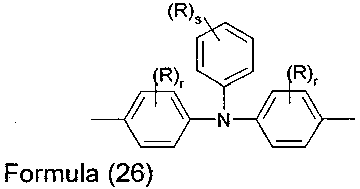 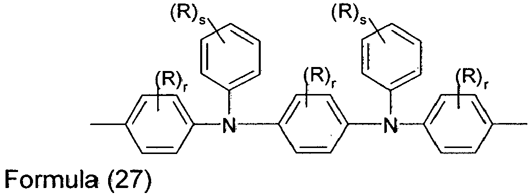 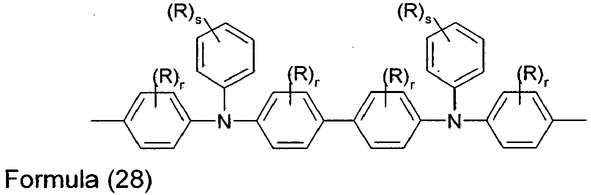 wherein
R which may be the same or different in each occurrence, is selected from H, substituted or unsubstituted aromatic or heteroaromatic group, alkyl, cycloalkyl,alkoxy, aralkyl, aryloxy, arylthio, alkoxycarbonyl, silyl, carboxy group, a halogen atom, cyano group, nitro group or hydroxy group,
r is 0, 1,2, 3 or 4, and
s is 0, 1, 2, 3, 4 or 5.

Further preferred polymeric HTM or HIM is a polymer comprising at least one of following repeat unit according to Formulae (29).

- (T¹)_{c} - (Ar⁴)_{d} - (T²)ₑ - (Ar⁵)_{f} - Formula (29)

wherein
T¹ and T² are independently of each other selected from thiophene, selenophene, thieno[2,3b]thiophene, thieno[3,2b]thiophene, dithienothiophene, pyrrole, aniline , all of which are optionally substituted with R⁵,
R⁵ is in each occurrence independently of each other selected from halogen, -CN, -NC, -NCO, -NCS, -OCN, SCN, C(=O)NR⁰R⁰⁰, -C(=O)X, - C(=O)R⁰, -NH₂, -NR⁰R⁰⁰, SH, SR⁰, -SO₃H, -SO₂R⁰, -OH, -NO₂, -CF₃, -SF₅, optionally substituted silyl, or carbyl or hydrocarbyl with 1 to 40 C atoms that is optionally substituted and optionally contains one or more hetero atoms,
Ar⁴ and Ar⁵ are independently of each other mononuclear or polynuclear aryl or heteroaryl, which is optionally substituted and optionally fused to the 2,3-positions of one or both of the adjacent thiophene or selenophene groups,
c and e are independently of each other 0, 1, 2, 3 or 4, with 1 < c + e ≤ 6,
d and f are independently of each other 0, 1, 2, 3 or 4.

Examples for polymeric HTMs are as disclosed in WO 2007131582 A1 and WO 2008/009343A1.

Preferably, the said polymer comprises units of groups 2, which are preferably selected from groups comprising the low molecular weight ETMs or EIMs as described above.

Further preferred units from group 2, which have electron-injection or electron-transport properties, are, for example, pyridine, pyrimidine, pyridazine, pyrazine, oxadiazole, quinoline, quinoxaline and phenazine derivatives, but also triarylboranes and further O, S, or N containing heterocycles.

Preferably, the said polymer comprises units from group 3 in which structures which increase the hole mobility and the electron mobility (i.e. units from groups 1 and 2) are bonded directly to one another. Some of these units may serve as emitters and shift the emission colour into the green, yellow or red. Their use is thus suitable, for example, for the production of other emission colours or a broad-band emission from originally blue-emitting polymers.

Preferably, the polymer comprises units of group 4, which are preferably selected from the groups comprising phosphorescent emitter, particularly emissive metal complexes as described above. Particular preference is given here to corresponding structural units which contain elements from groups 8 to 10 (Ru, Os, Rh, Ir, Pd, Pt).

Preferably, the said polymer comprises units of group 5, which can improve the transition from the singlet state to the triplet state and which, employed in support of the structural elements from group 4, improve the phosphorescence properties of these structural elements. Suitable for this purpose are, in particular, carbazole and bridged carbazole dimer units, as described in DE 10304819 A1 and DE 10328627 A1. Also suitable for this purpose are ketones, phosphine oxides, sulfoxides, sulfones, silane derivatives and similar compounds, as described in DE 10349033 A1. Further preferred structure units can be selected from groups comprising the low molecular weight phosphorescent matrices as described above.

Preferably, the said polymer comprises units of group 6, which influence the morphology and/or emission colour of the polymers, are, besides those mentioned above, those which have at least one further aromatic or another conjugated structure which do not fall under the above-mentioned groups, i.e. which have only little effect on the charge-carrier mobilities, which are not organometallic complexes or which have no influence on the singlet-triplet transition. Structural elements of this type may influence the morphology and/or emission colour of the resultant polymers. Depending on the unit, they can therefore also be employed as emitters. Preference is given here to aromatic structures having 6 to 40 C atoms or also tolan, stilbene or bisstyrylarylene derivatives, each of which may be substituted by one or more radicals R¹. Particular preference is given here to the incorporation of 1,4-phenylene, 1,4-naphthylene, 1,4- or 9,10-anthrylene, 1,6-, 2,7- or 4,9-pyrenylene, 3,9- or 3,10-perylenylene, 4,4'-biphenylylene, 4,4"-terphenylylene, 4,4' bi 1,1'-naphthylylene, 4,4'-tolanylene, 4,4'-stilbenylene or 4,4"-bisstyrylarylene derivatives.

Preferably, the said polymer comprises units of group 7 which contain aromatic structures having 6 to 40 C atoms which are typically used as polymer backbone. These are, for example, 4,5-dihydropyrene derivatives, 4,5,9,10-tetrahydropyrene derivatives, fluorene, derivatives as disclosed for example in US 5962631, WO 2006/052457 A2 and WO 2006/118345A1, 9,9'-spirobifluorene derivatives as disclosed for example in WO 2003/020790 A1, 9,10-phenanthrene derivatives as disclosed, for example, in WO 2005/104264 A1, 9,10-dihydrophenanthrene derivatives as disclosed for example in WO 2005/014689 A2, 5,7-dihydrodibenzooxepine derivatives and cis- and trans-indenofluorene derivatives as disclosed for example in WO 2004041901 A1, WO 2004113412 A2 and , binaphthylene derivatives as disclosed for example in WO 2006/063852 A1, and further units as disclosed for example in WO 2005/056633A1, EP 1344788A1 and WO 2007/043495A1, WO 2005/033174 A1, WO 2003/099901A1 and DE 102006003710.3.

Further preferred structural elements from group 7 are selected from fluorene derivatives, as disclosed for example in US 5,962,631, WO 2006/052457 A2 and WO 2006/118345 A1, spiro-bifluorene derivatives as disclosed for example in WO 2003/020790 A1, benzofluorene, dibenzofluorene, benzothiophene, dibenzofluorene and their derivatives as disclosed for example in WO 2005/056633A1, EP 1344788A1 and WO 2007/043495A1

Very preferred structural elements of group 7 are those of Formula (30): 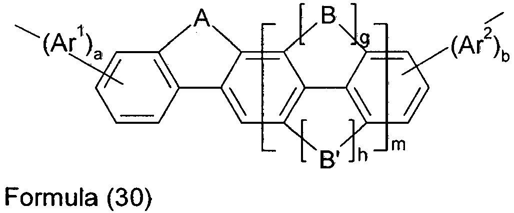 wherein
A, B and B' are independently of each other, and in case of multiple occurrence independently of one another, a divalent group, preferably selected from -CR¹R²-, -NR¹-, -PR¹-, -O-, -S-, -SO-, -SO₂- -CO-, -CS-,-CSe-, -P(=O)R¹-, -P(=S)R¹- and -SiR¹R²-,
R¹ and R² are independently of each other identical or different groups selected from H, halogen, -CN, -NC, -NCO, -NCS, -OCN, -SCN,-C(=O)NR⁰R⁰⁰, -C(=O)X, -C(=O)R⁰, -NH₂, -NR⁰R⁰⁰, -SH, -SR⁰, -SO₃H,-SO₂R⁰, -OH, -NO₂, -CF₃, -SF₅, optionally substituted silyl, or carbyl or hydrocarbyl with 1 to 40 C atoms that is optionally substituted and optionally comprises one or more hetero atoms, and optionally the groups R¹ and R² form a spiro group with the fluorene moiety to which they are attached,
X is halogen,
R⁰ and R⁰⁰ are independently of each other H or an optionally substituted carbyl or hydrocarbyl group optionally comprising one or more hetero atoms,
each g is independently 0 or 1 and each corresponding h in the same subunit is the other of 0 or 1,
m is an integer ≥ 1
Ar¹ and Ar² are independently of each other mono- or polynuclear aryl or heteroraryl that is optionally substituted and optionally fused to the 7,8-positions or 8,9-positions of the indenofluorene group,
a and b are independently of each other 0 or 1.

If the groups R¹ and R² form a spiro group with the fluorene group to which they are attached, it is preferably spirobifluorene.

The groups of Formula (30) are preferably selected from the following Formulae (31) to (35): 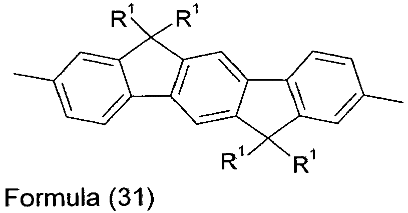 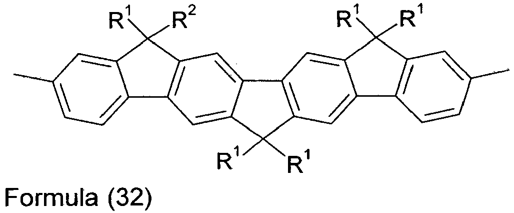 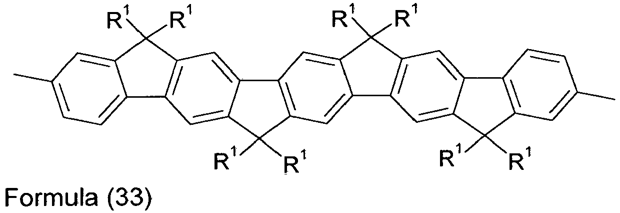 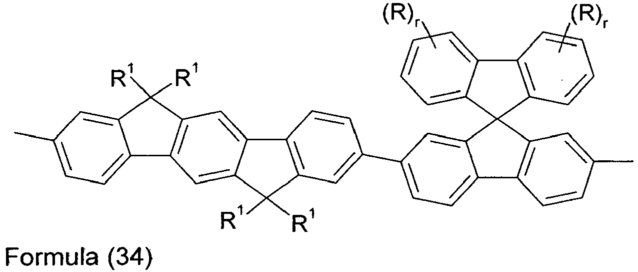 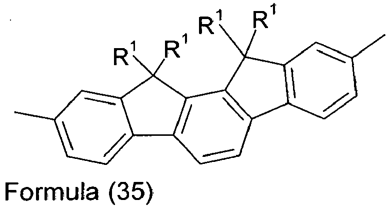 wherein R¹ is as defined in Formula (30), r is 0, 1, 2, 3 or 4, and R has one of the meanings of R¹.

R is preferably F, Cl, Br, I, -CN, -NO₂, -NCO, -NCS, -OCN, -SCN, - C(=O)NR⁰R⁰⁰, -C(=O)X⁰, -C(=O)R⁰, -NR⁰R⁰⁰, optionally substituted silyl, aryl or heteroaryl with 4 to 40, preferably 6 to 20 C atoms, or straight chain, branched or cyclic alkyl, alkoxy, alkylcarbonyl, alkoxycarbonyl, alkylcarbonlyoxy or alkoxycarbonyloxy with 1 to 20, preferably 1 to 12 C atoms, wherein one or more H atoms are optionally replaced by F or Cl, and wherein R⁰, R⁰⁰ and X⁰ are as defined above.

Particularly preferred groups of Formula (30) are selected from the following Formulae (36) to (39): 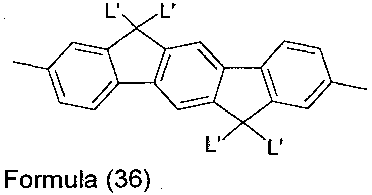 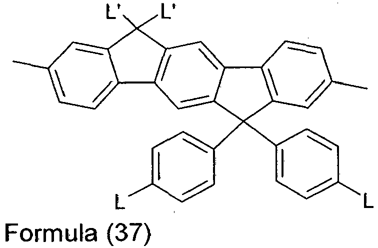 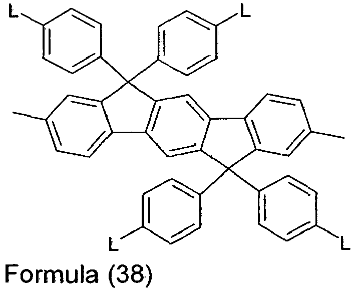 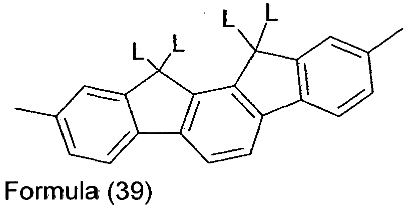 wherein
L is H, halogen or optionally fluorinated, linear or branched alkyl or alkoxy with 1 to 12 C atoms, and is preferably H, F, methyl, i-propyl, t-butyl, n-pentoxy, or trifluoromethyl, and
L' is optionally fluorinated, linear or branched alkyl or alkoxy with 1 to 12 C atoms, and is preferably n-octyl or n-octyloxy.

Preference is given to polymers suitable for use in the invention which simultaneously comprise one or more units selected from groups 1 to 8. It may likewise be preferred for more than one structural unit from a group to be present simultaneously.

Preference is given to polymers suitable for use in the invention which, besides structural units of an emitter, also comprise at least one structural unit from the above-mentioned groups. At least two structural units are particularly preferably from different classes of those mentioned above.

The proportion of the different classes of groups, if present in the polymer, is preferably in each case at least 5 mol%, particularly preferably in each case at least 10 mol%. In particular, one of these structural units is selected from the group of hole-conducting units and the other group is an emitting unit, where these two functions (hole conduction and emission) may also be taken on by the same unit.

However, a smaller proportion of the emitting units, in particular green- and red-emitting units, may also be preferred, for example for the synthesis of white-emitting copolymers. The way in which white-emitting copolymers can be synthesised is described in detail in DE 10343606 A1. In order to ensure adequate solubility, it is preferred for on average at least 2 non-aromatic C atoms to be present in the substituents per recurring unit. Preference is given here to at least 4 and particularly preferably at least 8 C atoms. In addition, individual C atoms of these may be replaced by O or S. However, it is entirely possible for this to mean that a certain proportion of recurring units does not carry any further non-aromatic substituents.

In order to avoid impairing the morphology of the film, it is preferred to have no long-chain substituents having more than 12 C atoms in a linear chain, particularly preferably none having more than 8 C atoms and in particular none having more than 6 C atoms.

The said polymer may be statistical or random copolymers, alternating or regioregular copolymers, block copolymers or combinations thereof.

In another preferred embodiment, the said polymer is a side-chain non-conjugated polymer, which is especially important for phosphorescent emission based on polymer. In general, such phosphorescent polymer is obtained by means of radical copolymerization of vinyl compounds, and comprises at least one phosphorescent emitter and at least one charge transport unit on side chain, as disclosed in US 7250226 B2. Further examples for such phosphorescent polymer are disclosed for example in JP 2007/211243 A2, JP 2007/197574 A2, US 7250226B2, JP 2007/059939A.

In a further preferred embodiment, the said polymer is a main-chain non-conjugated polymer, where the backbone units are connected by spacer on main-chain. Like side-chain non-conjugated polymer, main-chain non-conjugated polymers give also a high triplet level. An example for triplet OLEDs based on main-chain non-conjugated polymers is disclosed in DE 102009023154.4.

In a further embodiment, the said polymer can also be a non-conjugated polymer for fluorescent emission. Preferred singlet non-conjugated polymers are, for example, side-chain polymers with antracenenes, benzanthrecenes and their derivates in the side-chain, as disclosed in JP 2005/108556, JP 2005/285661, JP 2003/338375 etc..

The said polymers can also act as ETM or HTM, preferably the polymer is a non-conjugated polymer.

Typical ionic species, also called ionic materials, which are suitable for the composition of the devices according to the present invention, have the general formula K⁺A⁻, wherein K⁺ and A⁻ represent a cation and an anion, respectively.

Preferably the ionic materials are soluble in the same solvent as the organic emissive material. This easily allows the preparation of a mixture comprising the said emitter material(s) and the ionic material(s). Typically organic emissive materials are soluble in common organic solvents, such as toluene, anisole, chloroform.

Preferably, the said ionic material is solid at room temperature and particularly preferably, the said ionic material is solid at room temperature and getting softer between 30 to 37°C.

Preferably the said ionic species is a cation. Suitable inorganic cations K⁺ can be selected from, for example, K⁺(potassium) and Na⁺. Suitable organic cations K⁺ can be selected from ammonium-, phosphonium, thiouronium-, guanidinium cations as shown in Formulae (40) to (44) or heterocyclic cations as shown in Formulae (45) to (72). 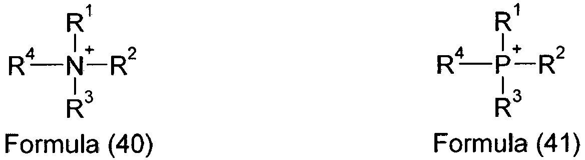 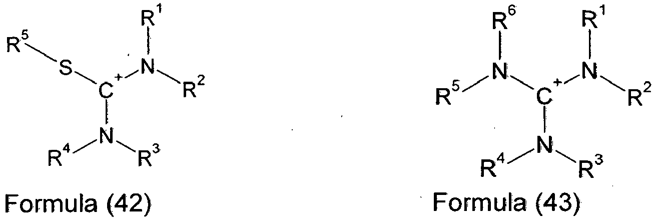 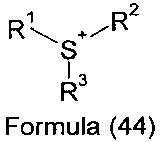 wherein
R¹ to R⁶ can be, independently from each other, selected from linear or hyperbranched alkyl rests with 1 to 20 C-atoms, linear or hyperbranched alkenyl rests with 2 to 20 C-atoms and one or more non-conjugated double bonds, linear or hyperbranched alkinyl rests with 2 to 20 C-atoms and one or more non-conjugated triple bond, saturated, partly saturated or completely saturated cycloalkyl with 3 to 7 C-atoms, which can further be substituted with alkyl groups having 1 to 6 C-atoms, wherein one or more substituents R may be partly or completely substituted with halogen, particularly with -F and/or -Cl, or partly substituted with -OR', -CN,-C(O)OH, -C(O)NR'₂, -SO₂NR'₂, -SO₂OH, -SO₂X, -NO₂, wherein one or two non adjacent and non α-carbon atoms of R¹ to R⁶ can be substituted with groups selected from -O-, -S-, -S(O)-, -SO₂-, -N⁺R'₂⁻, -C(O)NR'-, -SO₂NR'-, and -P(O)R'-, wherein R' = H, unsubstituted, partly or completely with -F substituted C1 to C6-alkyl, C3 to C7-cycloalkyl, unsubstituted or substituted phenyl and X =halogen.

In Formula (40) R¹ to R⁴ can be H, with the provision that at least one of the rests R¹ to R⁴ is not H. In Formula (41) R¹ to R⁴ can be H and NR'₂, wherein R' is defined as above. In Formula (42) R¹ to R⁵ can be H. In Formula (43) R¹ to R⁶ can be H, CN, and NR'₂, wherein R' is defined as above.

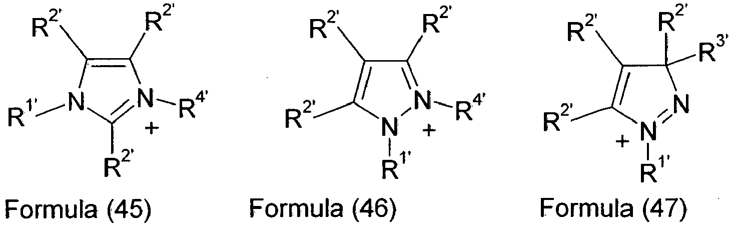

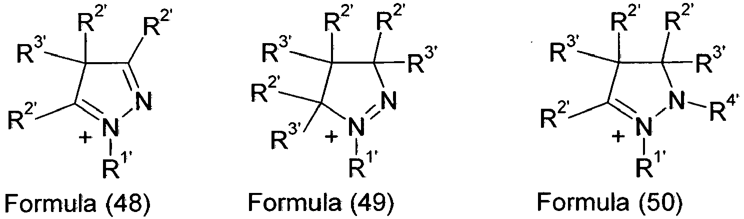

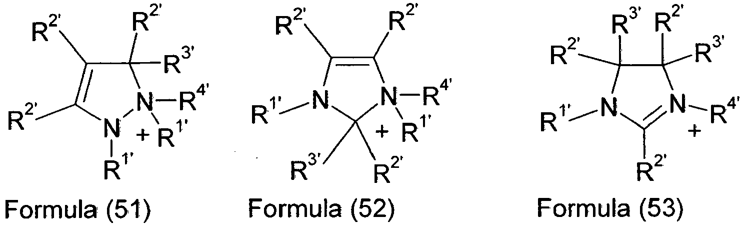

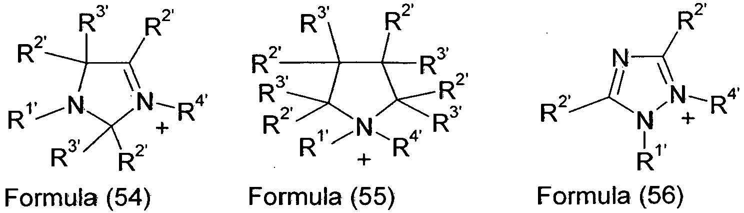

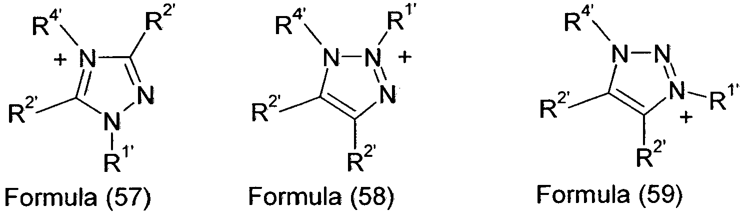

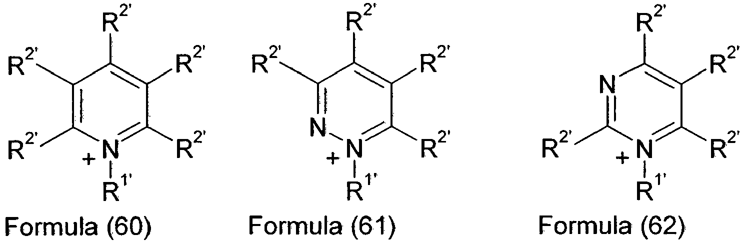

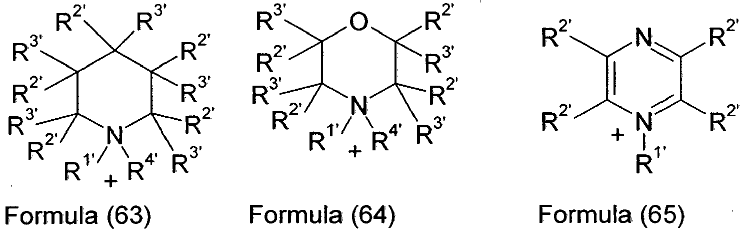

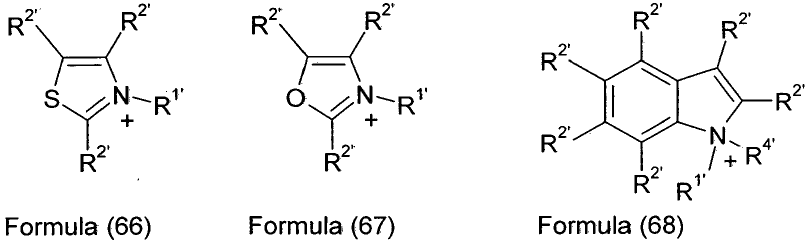

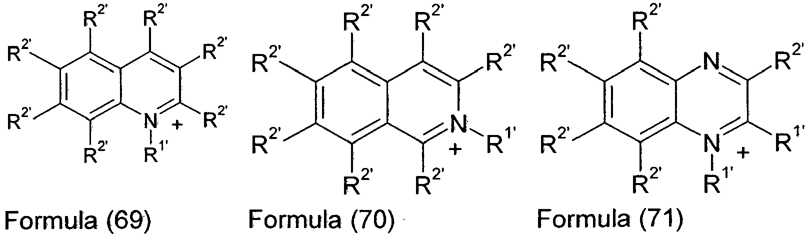

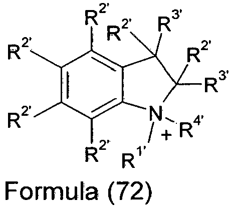

Wherein the substituents R¹' to R⁴' are independently from each other selected from H, CN, linear and branched alkyl rest with 1 to 20 C-atoms, linear or branched alkenyl rest with 2 to 20 C-atoms and one or more non conjugated double bonds, linear or branched alkinyl rest with 2 to 20 C-atoms and one or more non conjugated triple bonds, partly or completely non saturated cycloalkyl rest with 3 to 7 C-atoms which can be substituted with alkyl rests with 1 to 6 C-atoms, saturated and partly or completely non saturated heteroaryls, heteroaryl-C₁-C₆-alkyl, or alkyl-C₁-C₆-alkyl, wherein the substituents R¹', R²', R³' and/or R⁴' together can form a ring, wherein one or more of the substituents R¹' to R⁴' can partly or completely be substituted with halogen, particularly with -F and/or -Cl, and -OR', -CN, -C(O)OH, - C(O)NR'₂, -SO₂NR'₂, -C(O)X, -SO₂OH, -SO₂X, -NO₂, wherein the substituents R¹' und R⁴' are not substituted with halogen at the same time, wherein one or two carbon atoms of the substituents R¹' and R²', which are non adjacent or bound to an heteroatom, can be substituted by a group selected from -O-, -S-, -S(O)-, -SO₂-, -N⁺R'₂-, -C(O)NR'-,-SO₂NR'-, and -P(O)R'- wherein R' = H, unsubstituted, partly or completely with -F substituted alkyl with 1 to 6 C-atoms, cycloalkyl with 3 to 7 C-atoms, unsubstituted or substituted phenyl and X =halogen.

Preference is given to R²' selected from -OR',-NR'₂, -C(O)OH, -C(O)NR'₂, -SO₂NR'₂) -SO₂OH, -SO₂X, and -NO₂.

Further preferred ionic materials are disclosed in, e.g., US 2007/0262694 A1.

Further particularly preferred ionic materials comprise a cation having a structure represented by Formula (73). They include N,N,N-trimethylbutyl ammonium ion, N-ethyl-N,N-dimethyl-propyl ammonium ion, N-ethyl-N,N-dimethylbutyl ammonium ion, N,N,-dimethyl-N-propylbutyl ammonium ion, N-(2-methoxyethyl)-N,N-dimethylethyl ammoniumion, 1-ethyl-3-methyl imidazolium ion, 1-ethyl-2,3-dimethyl imidazoliun ion, 1-ethyl-3,4-dimethyl imidazolium ion, 1-ethyl-2,3,4-trimethyl imidazolium ion, 1-ethyl-2,3,5-trimethyl imidazolium ion, N-methyl-N-propyl pyrrolidinium ion, N-butyl-N-methyl pyrrolidinium ion, N-sec-butyl-N-methylpyrrolidinium ion, N-(2-methoxyethyl)-N-methylpyrrolidinium ion, N-(2-ethoxyethyl)-N-methylpyrrolidinium ion, N-methyl-N-propyl piperidinium ion, N-butyl-N-methyl pipridinium ion, N-sec-butyl-N-methylpiperidinium ion, N-(2-methoxyethyl)-N-methyl piperidiniumion and N-(2-ethoxyethyl)-N-methyl piperidinium ion.

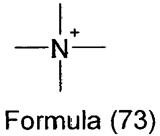

Very particularly preferred is N-methyl-N-propyl piperidinium.

Particularly preferred ionic material is a compound selected from the group of ionic compounds, which are soluble in common organic solvents such as toluene, anisole, and chloroform, consisting of methyltrioctylammonium trifluoromethane-sulfonate (MATS), 1-methyl-3-octylimidazolium octylsulfate, 1-butyl-2,3-dimethylimidazolium octylsulfate, 1-octadecyl-3-methylimidazolium bis(trifluoromethylsulfonyl)imide, 1-octadecyl-3-methylimidazolium tris(pentafluoroethyl)trifluorophosphate, 1,1-dipropylpyrrolidimium bis(trifluoromethylsulfonyl)imide, trihexyl(tetradecyl)phosphonium bis(1,2-bezenediolato(2-)-O,O')borate, and N,N,N',N',N',N'-pentamethyl-N'-propylguanidinium trifluoromethanesulfonate.

Further preferred cations are selected from compounds of one of the general Formulae (74) to (79)

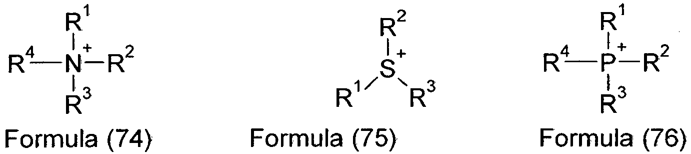

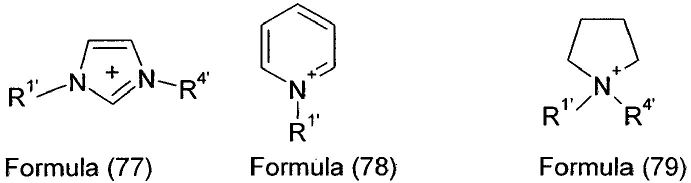

Wherein R¹ to R⁴ are defined as in Formulae (40), (41), and (44), and R¹' and R⁴' as in Formulae (45), (60), and (55).

Further preferred ionic materials suitable for the composition and device according to the present invention is a compound wherein one of K⁺ or A⁻ is covalently bounded to a polymer backbone.

Further preferred ionic materials suitable for the composition and device according to the present invention are selected from compounds wherein one of K⁺ or A⁻ is an organic emissive material, which can be selected from small molecule and polymeric emissive materials as described elsewhere within the present invention.

Preferably the said ionic species is an anion. Suitable anions A⁻ can be selected from [HSO₄]⁻, [SO₄]²⁻, [N₃]⁻,[BF₄]⁻, [(R_{F})BF3]⁻, [(R_{F})₂BF₂]⁻, [(R_{F})₃BF]⁻, [(R_{F})₄B]⁻, [B(CN)₄]⁻, [PO₄]³⁻, [HPO₄]²⁻, [H₂PO₄]⁻, [Alkyl-OPO₃]²⁻, [(Alkyl-O)₂PO₂]⁻, [Alkyl-PO₃]²⁻, [R_{F}PO₃]²⁻, [(Alkyl)₂PO₂]⁻, [(R_{F})₂PO₂]⁻, [R_{F}SO₃]⁻, [HOSO₂(CF₂)ₙSO₂O]⁻, [OSO₂(CF₂)ₙSO₂O]²⁻, [Alkyl-SO₃]⁻, [HOSO₂(CH₂)ₙSO₂O]⁻, [OSO₂(CH₂)ₙSO₂O]²⁻, [Alkyl-OSO₃]⁻, [Alkyl-C(O)O]⁻, [HO(O)C(CH₂)ₙC(O)O]⁻, [R_{F}C(O)O]⁻, [HO(O)C(CF₂)ₙC(O)O]⁻, [O(O)C(CF₂)ₙC(O)O]²⁻, [(R_{F}SO₂)₂N]⁻, [(FSO₂)₂N]⁻, [((R_{F})₂P(O))₂N]⁻, [(R_{F}SO₂)₃C]⁻, [(FSO₂)₃C]⁻, Cl⁻ and/or Br⁻
wherein:
n = 1 to 8;
R_{F} is fluorinated alkyl of formula (CₘF₂ₘ₋ₓ₊₁Hₓ) with m = 1 to 12 and x = 0 to 7, wherein for m = 1 and x = 0 to 2, and/or fluorinated (also perfluorinated) aryl or alkyl-aryl.

The alkyl-group mentioned above can be selected from linear or hyperbranched alkyl groups with 1 to 20 C-atoms, preferably with 1 to 14 C-atoms and particularly preferably with 1 to 4 C-atoms. Preferably R_{F} means CF₃, C₂F₅, C₃F₇ or C₄F₉.

Preferred anions are selected from PF₆⁻, [PF₃(C₂F₅)₃]⁻, [PF₃(CF₃)₃]⁻, BF₄⁻, [BF₂(CF₃)₂]⁻, [BF₂(C₂F₅)₂]⁻, [BF₃(CF₃)]⁻, [BF₃(C₂F₅)]⁻, [B(COOCOO)₂⁻ (BOB⁻), CF₃SO₃⁻ (Tf⁻), C₄F₉SO₃ (Nf), [(CF₃SO₂)₂N]⁻ (TFSI⁻), [(C₂F₅SO₂)₂N]⁻ (BETI⁻), [(CF₃SO₂)(C₄F₉SO₂)N]⁻, [(CN)₂N]⁻ (DCA⁻), [CF₃SO₂]₃C]⁻, and [(CN)₃C]⁻.

Further preferred ionic materials suitable for the composition of the device according to the present invention selected from compounds with the formula (Kⁿ⁺)ₐ(A^{m-})_{b}, wherein n, m, a, and b are integers from 1 to 3, and n × a - m × b = 0 and wherein one of Kⁿ⁺ or A^{m-} is an organic emissive material, which can be selected from compound comprising groups of small molecule or polymeric emitters as outlined elsewhere within the present invention. Preferably, n. m a, b are 1.

One particular advantage of such composition is that no additional ionic compound is needed.

In a preferred embodiment, in the said compound in form of (Kⁿ⁺)ₐ(A^{m-})_{b}, one of Kⁿ⁺ or A^{m-} is an emissive metal complex, and particularly preferably Kⁿ⁺ is an emissive metal complex, wherein the metal can be selected from transition metals, preferably those of group **VIII** elements, lanthanides, and actinides, particularly preferably selected from Rh, Os, Ir, Pt, Au, Sm, Eu, Gd, Tb, Dy, Re, Cu, W, Mo, Pd, Ag, Ru, and very particularly preferably selected from Ru, Os, Ir, Re. Some non-limiting examples for Kⁿ⁺ are [Ir(PPY)₂(bPY)]⁺, [Ir(ppy)₂(dpp)]⁺, [Ir(ppy)₂(phen)]⁺, [Ru(bpy)₃]²⁺, [Os(bpy)₂L)]²⁺ (L = cis-1,2-bis(diphenylphosphino)ethylene).

In a further embodiment of the present invention the said composition comprises a compound with the formula (Kⁿ⁺)ₐ(A^{m-})_{b}, wherein one of Kⁿ⁺ or A^{m-} is an emissive singlet emitter, and particularly preferably Kⁿ⁺ an emissive singlet emitter. Such kind of compound can be selected from charged laser dyes, for examples p-quaterphenyl-4,4"'-disulfonicacid disodiumsalt (polyphenyl 1), p-quaterphenyl-4,4"'-disulfonicacid dipotassiumsalt (polyphenyl 2), 2-(4-biphenylyl)-6-phenylbenzoxazotetrasulfonicacid potassium salt (furan 2), [1,1'-biphenyl]-4-sulfonic acid, 4',4"-1,2-ethene-diylbis-, dipotassium salt (stilbene 1), 2,2'-([1,1'-biphenyl]-4,4'-diyldi-2,1-ethenediyl)-bis-benzenesulfonic acid disodium salt (stilbene 3), benzofuran,2,2'-[1,1'-biphenyl]-4,4'-diyl-bis-tetrasulfonic acid (tetrasodium salt) (furan 1), 2-(p-dimethylaminostyryl)-pyridylmethyl Iodide (DASPI), 2-(p-dimethylaminostyryl)-benzothiazolylethyl Iodide (DASBTI), 3,3'-diethyloxacarbocyanine Iodide (DOCI), 4,4-difluoro-1,3,5,7,8-pentamethyl-4-bora-3a,4a-diaza-s-indacene 1,3,5,7,8-pentamethylpyrromethenedifluoroborate complex (pyrromethene 546), 3,3'-dimethyl-9-ethylthiacarbocyanine Iodide (DMETCI), disodium-1,3,5,7,8-pentamethylpyrromethene-2,6-disulfonate-difluoroborate complex (pyrromethene 556), 4,4-difluoro-2,6-diethyl-1,3,5,7,8-pentamethyl-4-bora-3a,4a-diaza-s-indacene 2,6-diethyl-1,3,5,7,8-pentamethylpyrromethenedifluoroborate complex (pyrromethene 567), o-(6-amino-3-imino-3H-xanthen-9-yl)-benzoic acid (rhodamine 110), benzoic acid, 2-[6-(ethylamino)-3-(ethylimino)-2,7-dimethyl-3H-xanthen-9-yl], perchlorate (rhodamine 19), 4,4-difluoro-2,6-di-n-butyl-1,3,5,7,8-pentamethyl-4-bora-3a,4a-diaza-s-indacene 2,6-di-n-butyl-1,3,5,7,8-pentamethylpyrromethenedifluoroborate complex (pyrromethene 580), benzoic acid, and 2-[6-(ethylamino)-3-(ethylimino)-2,7-dimethyl-3H-xanthen-9-yl]- ethyl ester, monohydrochloride (rhodamine 6G), which are commercially available at Lambda Physik AG, Goettingen, Germany.

Preferably the composition comprises at least one compound of the formula (Kⁿ⁺)ₐ(A^{m-})_{b}, characterized in that one of Kⁿ⁺ or A^{m-} is an emissive singlet emitter.

Very preferably Kⁿ⁺ is an emissive singlet emitter. Kⁿ⁺ is preferably selected from the group as defined above.

The device according to claim 1 is an electroluminescent device. Preference is given to a device comprising said composition comprising 3, particularly preferably 2, and very particularly preferably 1 compound of said formula (Kⁿ⁺)ₐ(A^{m-})_{b}.

The said device comprises at least two electrodes. Preferably it comprises two electrodes, a cathode and an anode. In a preferred embodiment both electrodes are connected by said composition.

Preferably the device comprising a composition comprising a compound of formula (Kⁿ⁺)ₐ(A^{m-})_{b}

Further the compositions may also comprise at least one host material. Host materials are usually used in combination with emitter and have, in general, larger energy gaps between the HOMO and the LUMO as compared to emitter materials. In addition, host materials behave either as electron or hole transport material. Host materials can also have both electron and hole transport properties. In case singlet transitions are predominantly responsible for luminescence in OLECs, a maximal overlap between the absorption spectrum of the emitter with the photoluminescence spectrum of the host material is desirably. This ensures the energy transfer from the host material to the emitter.

A host material is also called matrix or matrix material, particularly if a host is meant which is used in combination with a phosphorescent emitter. In the case of a copolymer comprising emitter units, the polymer backbone acts as a host.

In principle any host material known to one skilled in the art can be employed according to the present invention. Depending on the kind of emitter employed host materials can be separated into two categories, hosts for fluorescent emitter and hosts for phosphorescent emitter (also called matrix material).

Preference is given to host materials selected from anthracenes, benzanthracenes, indenofluorenes, fluorenes, spirobifluorenes, phenanthrenes, dehydrophenanthrenes, thiophenes, triazines, imidazole, ketones, carbazoles, indolocarbazoles, indenocarbazoles, triarylamines, and derivatives thereof.

Particular preference is given to host materials selected from anthracenes, benzanthracenes, indenofluorenes, fluorenes, spirobifluorenes, phenanthrenes, dehydrophenanthrenes, thiophenes, triazines, imidazole, ketones, carbazoles, indolocarbazoles, indenocarbazoles, and triarylamines.

The composition of the device according to the present invention may also comprise more than one host material, preferably it comprises 3 host materials, particularly preferably it comprises 2 host materials, and very particularly preferably one host material. If the composition comprises at least two host materials, the host materials are also referred to as co-hosts or co-host materials.

Preferred host materials suitable for fluorescent emitter are selected from anthracenes, benzanthracenes, indenofluorenes, fluorenes, spirobifluorenes, phenanthrenes, dehydrophenanthrenes, thiophenes, triazines, imidazole indolocarbazoles, indenocarbazoles, and derivatives thereof.

Preferred host materials suitable for fluorescent emitter are selected from anthracenes, benzanthracenes, indenofluorenes, fluorenes, spirobifluorenes, phenanthrenes, dehydrophenanthrenes, thiophenes, triazines, imidazole, indolocarbazoles, and indenocarbazoles.

Particularly preferred host materials for fluorescent emitter are selected from the classes of the oligoarylenes (for example 2,2',7,7'-tetraphenyl-spirobifluorene in accordance with EP 676461 or dinaphthylanthracene), in particular the oligoarylenes containing condensed aromatic groups, such as, for example, phenanthrene, tetracene, coronene, chrysene, fluorene, spirofluorene, perylene, phthaloperylene, naphthaloperylene, deca-cyclene, rubrene, the oligoarylenevinylenes (for example 4,4'-bis(2,2-di-phenylethenyl)-1,1'-biphenyl (DPVBi) or 4,4-bis-2,2-diphenylvinyl-1,1-spirobiphenyl (spiro-DPVBi) in accordance with EP 676461), the polypodal metal complexes (for example in accordance with WO 2004/081017), in particular metal complexes of 8 hydroxyquinoline, for example aluminium(III) tris(8-hydroxyquinoline) (aluminium quinolate, Alq₃) or bis(2-methyl-8-quinolinolato)-4-(phenylphenolinolato)aluminium, also with imidazole chelate (US 2007/0092753 A1) and quinoline-metal complexes, amino-quinoline-metal complexes, benzoquinoline-metal complexes, the hole-conducting compounds (e.g. in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (e.g. WO 2006/117052) or the benzanthracenes (e.g. DE 102007024850). Particularly preferred host materials are selected from the classes of the oligoarylenes, containing naphthalene, anthracene, benzanthracene and/or pyrene, or atropisomers of these compounds, the ketones, the phosphine oxides and the sulfoxides. Very particularly preferred host materials are selected from the classes of the oligoarylenes, containing anthracene, benzanthracene and/or pyrene, or atropisomers of these compounds. For the purposes of this invention, an oligoarylene is intended to be taken to mean a compound in which at least three aryl or arylene groups are bonded to one another.

Further preferred host materials for fluorescent emitter are selected, in particular, from compounds of the Formula 80

Ar⁴⁻(Ar⁵)ₚ-Ar⁶ Formula 80

wherein
Ar⁴, Ar⁵, Ar⁶ are on each occurrence, identically or differently, an aryl or heteroaryl group having 5 to 30 aromatic ring atoms, which may be substituted by one or more radicals and
p is 1, 2, or 3,
the sum of the π-electrons in Ar⁴, Ar⁵ and Ar⁶ is at least 30 if p = 1 and is at least 36 if p = 2 and is at least 42 if p = 3.

It is particularly preferred in the host materials of the Formula 80 for the group Ar⁵ to stand for anthracene, which may be substituted by one or more radicals R¹, and for the groups Ar⁴ and Ar⁶ to be bonded in the 9 and 10-positions. Very particularly preferably, at least one of the groups Ar⁴ and/or Ar⁶ is a condensed aryl group selected from 1- or 2-naphthyl, 2-, 3-or 9-phenanthrenyl or 2-, 3-, 4-, 5-, 6- or 7-benzanthracenyl, each of which may be substituted by one or more radicals R¹. Anthracene-based compounds are described in US 2007/0092753 A1 and US 2007/0252517 A1, for example 2-(4-methylphenyl)-9,10-di-(2-naphthyl)anthracene, 9-(2-naphthyl)-10-(1,1'-biphenyl)anthracene and 9,10-bis[4-(2,2-diphenyl-ethenyl)phenyl]anthracene, 9,10-diphenylanthracene, 9,10-bis(phenyl-ethynyl)anthracene and 1,4-bis(9'-ethynylanthracenyl)benzene. Preference is also given to host materials containing two anthracene units (US 2008/0193796 A1), for example 10,10'-bis[1,1',4',1"]terphenyl-2-yl-9,9'-bisanthracenyl.

Further preferred host materials are derivatives of arylamine, styrylamine, fluorescein, perynone, phthaloperynone, naphthaloperynone, diphenyl-butadiene, tetraphenylbutadiene, cyclopentadienes, tetraphenylcyclopentadiene, pentaphenylcyclopentadiene, coumarine, oxadiazole, bis-benzoxazoline, oxazone, pyridine, pyrazine, imine, benzothiazole, benzoxazole, benzimidazole (US 2007/0092753 A1), for example 2,2',2"-(1,3,5-phenylene)tris[1-phenyl-1H-benzimidazole], aldazines, stilbene, styrylarylene derivatives, for example 9,10-bis[4-(2,2-diphenylethenyl)-phenyl]anthracene, and distyrylarylene derivatives (US 5121029), diphenylethylene, vinylanthracene, diaminocarbazole, pyran, thiopyran, diketopyrrolopyrrole, polymethine, mellocyanine, acridone, quinacridone, cinnamic acid esters and fluorescent dyes.

Particular preference is given to derivatives of arylamine and styrylamine, for example 4,4'-bis[N-(1-naphthyl)-N-(2-naphthyl)amino]biphenyl (TNB).

Preferred compounds with oligoarylene as hosts for fluorescent emitter are compounds as disclosed in, e.g., US 2003/0027016 A1, US 7326371 B2, US 2006/043858 A, US 7326371 B2, US 2003/0027016 A1,
WO 2007/114358, WO 2008/145239, JP 3148176 B2, EP 1009044,
US 2004/018383, WO 2005/061656 A1, EP 0681019B1,
WO 2004/013073A1, US 5077142, WO 2007/065678, and
US 2007/0205412 A1. Particularly preferred oligoarylene-based compounds are compounds having the Formulae (81) to (87).

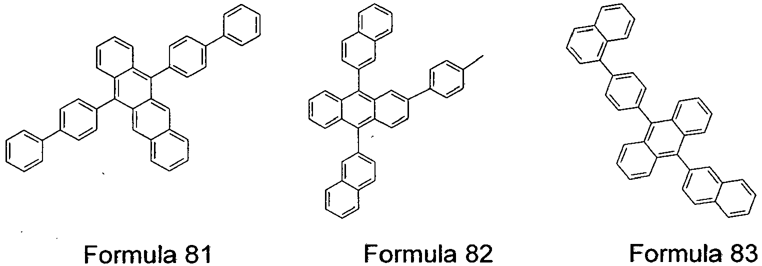

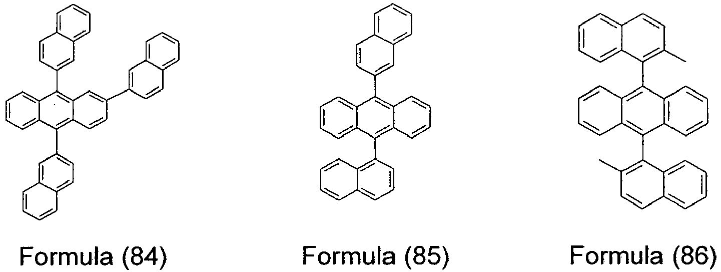

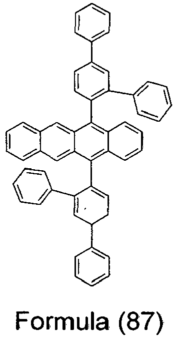

Further host materials for fluorescent emitter can be selected from spirobifluorene and derivates thereof, for example Spiro-DPVBi as disclosed in EP 0676461 and indenofluorene as disclosed in US 6562485.

The preferred host materials for phosphorescent emitter, i.e. matrix materials, are selected from ketones, carbazoles, indolocarbazoles, triarylamines, indenofluorenes, fluorenes, spirobifluorenes, phenathrenes, dehydrophenanthrenes, thiophenes, triazines, imidazoles and their derivatives. Some preferred derivatives are described below in more details.

If a phosphorescent emitter is employed, e.g. as electroluminescent component, the host material must fulfil rather different characteristics as compared to host materials used for fluorescent emitter. The host materials used for phosphorescent emitter are required to have a triplet level which is higher in energy as compared to the triplet level of the emitter. The host material can either transport electrons or holes or both of them. In addition, the emitter is supposed to have large spin-orbital coupling constants in order to facilitate singlet-triplet mixing sufficiently. This can be enabled by using metal complexes.

Preferred matrix materials are N,N-biscarbazolylbiphenyl (CBP), carbazole derivatives (e.g. WO 2005/039246, US 2005/0069729, JP 2004/288381, EP 1205527 or DE 102007002714), azacarbazoles (e.g. EP 1617710, EP 1617711, EP 1731584, JP 2005/347160), ketones (for example in accordance with WO 2004/093207), phosphine oxides, sulfoxides and sulfones (for example in accordance with WO 2005/003253), oligophenyl-enes, aromatic amines (e.g. US 2005/0069729), bipolar matrix materials (for example in accordance with WO 2007/137725), silanes (for example in accordance with WO 2005/111172), 9,9-diarylfluorene derivatives (e.g. in accordance with DE 102008017591), azaboroles or boronic esters (for example in accordance with WO 2006/117052), triazole derivatives, oxazoles and oxazole derivatives, imidazole derivatives, polyarylalkane derivatives, pyrazoline derivatives, pyrazolone derivatives, distyrylpyrazine derivatives, thiopyran dioxide derivatives, phenylenediamine derivatives, tertiary aromatic amines, styrylamines, indoles, anthrone derivatives, fluorenone derivatives, fluorenylidenemethane derivatives, hydrazone derivatives, silazane derivatives, aromatic dimethylidene compounds, porphyrin compounds, carbodiimide derivatives, diphenylquinone derivatives, phthalocyanine derivatives, metal complexes of 8 hydroxyquinoline derivatives, such as, for example, Alq₃, the 8 hydroxyquinoline complexes may also contain triarylaminophenol ligands as disclosed in US 2007/0134514, various metal complex-polysilane compounds with metal phthalocyanine, benzoxazole or benzothiazole as ligand, hole-conducting polymers, such as, for example, poly(N-vinylcarbazole) (PVK), aniline copolymers, thiophene oligomers, polythiophenes, polythiophene derivatives, polyphenylene derivatives, polyfluorene derivatives.

Further particularly preferred matrix materials are selected from compounds comprising indolocarbazoles and their derivatives (e.g. Formulae (88) to (94)), as disclosed for examples in DE 102009023155.2, EP 0906947B1, EP 0908787B1, EP 906948B1, WO 2008/056746A1, WO 2007/063754A1, WO 2008/146839A1, and WO 2008/149691A1.

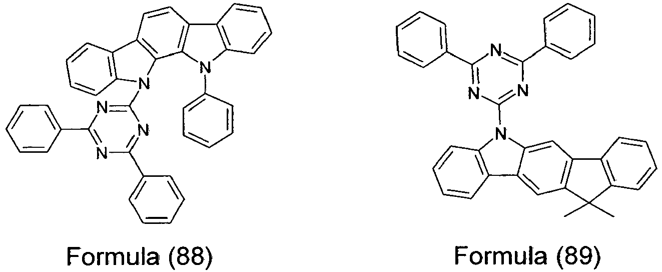

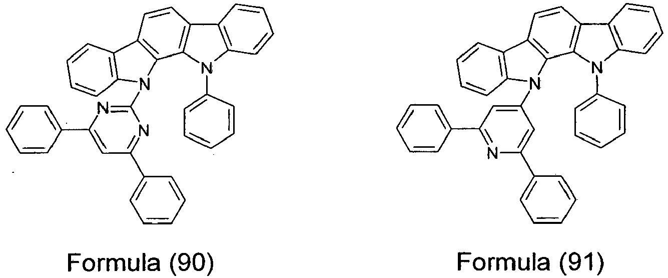

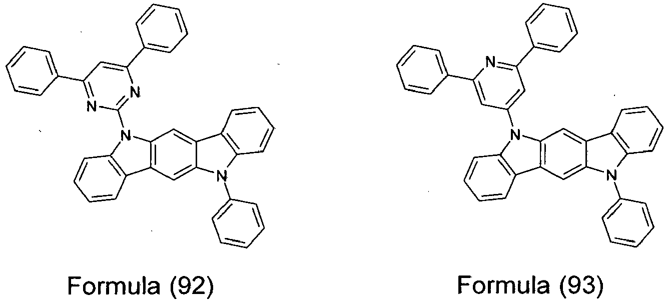

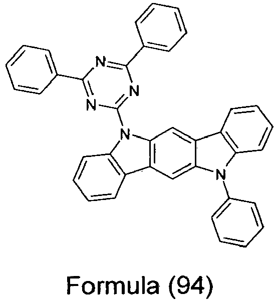

Examples of preferred carbazole derivatives are, 1,3-N,N-dicarbazolebenzene (= 9,9'-(1,3-phenylene)bis-9H-carbazole) (mCP), 9,9'-(2,2'-dimethyl[1,1'-biphenyl]-4,4'-diyl)bis-9H-carbazole (CDBP), 1,3-bis(N,N'-dicarbazole)benzene (= 1,3-bis(carbazol-9-yl)benzene), PVK (polyvinyl-carbazole), 3,5-di(9H-carbazol-9-yl)biphenyl and compounds of the Formulae (95) to (99).

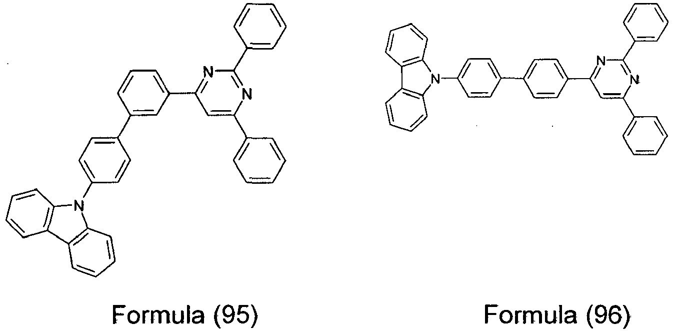

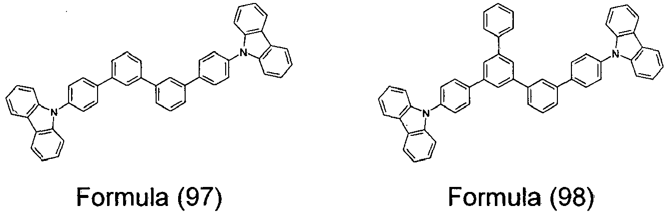

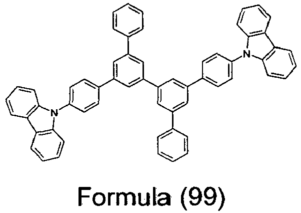

Preferred Si tetraaryl compounds are, for example, (US 2004/0209115, US 2004/0209116, US 2007/0087219 A1, US 2007/0087219 A1) the compounds of the Formulae (100) to (105).

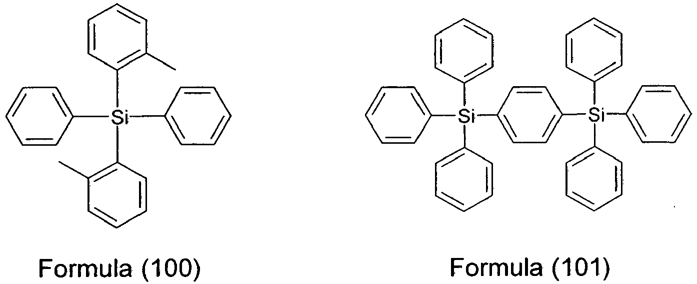

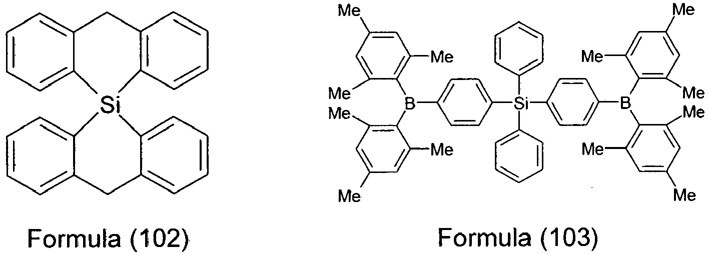

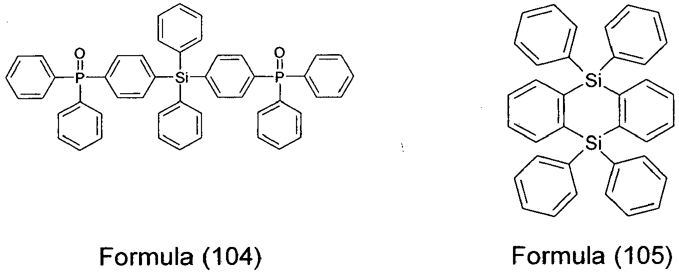

A particularly preferred matrix for phosphorescent dopants is the compound of Formula (106) (EP 652273 B1)

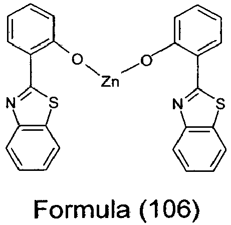

Further particularly preferred matrix materials for phosphorescent dopants are selected from compounds of the general Formula (107) (EP 1923448A1).

[M(L)₂]ₙ Formula (107)

wherein M, L, and n are defined as in the reference. Preferably M is Zn, and L is quinolinate, and n is 2, 3 or 4. Very particularly preferred are [Znq2]2, [Znq₂]₃, and [Znq2]4.

Preference is given to co-hosts selected from metal oxinoid complexes whereby lithium quinolate (Liq) or Alq₃ are particularly preferred.

The devices according to claim 1 can be used for the treatment and/or prophylaxis of skin diseases and/or-skin related conditions.

In a preferred embodiment the device of the present invention is used for the treatment and/or prophylaxis of skin diseases and/or-skin related conditions.

Skin as used herein is defined as the largest organ of the integumentary system including hair, scales, feathers and nails. The term skin also includes the tongue, mucosa and gingival.

In principle any therapeutic and cosmetic condition that is approachable by phototherapy can be treated. The distinction between the terms therapeutic and cosmetic depends, as outlined above, on individual circumstances, the severity of the condition and the assessment of the physician. As outlined in this invention many therapeutic conditions are associated with cosmetic effects, independent of the severity of the therapeutic disease.

The skin diseases and skin related conditions include, but are not limited to acneiform eruptions, autoinflammatory skin diseases or conditions, chronic blistering, conditions of the mucous membranes, conditions of the skin appendages, conditions of the subcutaneous fat, connective tissue diseases, abnormalities of dermal fibrous and elastic tissue, dermal and subcutaneous growths, dermatitis, atopic dermatitis, contact dermatitis, eczema, pustular dermatitis, seborrheic dermatitis and eczema, disturbances of pigmentation, drug eruptions, endocrine-related diseases and conditions, epidermal nevi diseases and conditions, neoplasms, cysts, erythemas, genodermatoses, infection-related diseases and conditions, bacterium-related diseases and conditions, mycobacterium-related diseases and conditions, mycosis-related diseases and conditions, parasitic infestations, stings, and bites, virus-related diseases and conditions, lichenoid eruptions, lymphoid-related diseases and conditions, melanocytic nevi and neoplasms, monocyte- and macrophage-related diseases and conditions, mucinoses, neurocutaneous, noninfectious immunodeficiency-related diseases and conditions, nutrition-related diseases and conditions, papulosquamous hyperkeratotic related diseases and conditions, pruritic related diseases and conditions, psoriasis (mild, mild to severe, and severe), reactive neutrophilic diseases and conditions, recalcitrant palmoplantar eruptions, diseases and conditions resulting from errors in metabolism, diseases and conditions resulting from physical factors, urticaria and angioedema, vascular-related diseases and conditions, and periodontitis or other diseases and conditions of the gingival.

Skin related diseases and conditions also include skin tumors, pre-malignant tumors, malignant tumors, cell carcinomas, secondary metastasis, radiodermatitis and keratosis.

The healing of wounds can also be assigned to skin diseases and skin related conditions. Wound healing can, hereby, occur at the outer surface of the subject to be treated, at its internal parts, at the skin, eye, nail or nail bed, any surface in the subject's mouth, and at the mucosa, gingival, epithelial surface of the vascular system or other part of the subjects body.

The devices according to the present invention can also be used in cosmetics for skin care and skin repair, e.g. as light plaster. The wavelengths or range of wavelengths emitted by said compositions of the devices is in the range between 400 and 800 nm, preferably between 450 and 750 nm, particularly preferably between 500 and 700 nm, and very particularly preferably between 580 and 640nm.

Preferred skin diseases and skin-related conditions are selected from acne, psoriasis, eczema, edema, dermatitis, atopic dermatitis, vitiligo, Bowens disease, tumors, pre-malignant tumors, malignant tumors, basal cell carcinomas, squamous cell carcinomas, secondary metastases, cutaneous T-cell lymphomas, solar keratosis, arsenical keratosis, radiodermatitis, and cellulite

Further preferred skin diseases and skin-related conditions are selected from psoriasis, polymorphous light eruption, solar urticaria, actinic reticuloid atopic eczema, vitiligo, pruritus, lichen planus, early cutaneous T-cell lymphoma, dermographism, and pityriasis lichenoides. Preferably these diseases and conditions are treated with light having a wavelength or a range of wavelengths between 200 and 500 nm, particularly preferably between 250 and 400 nm, and very particularly preferably between 270 and 350 nm.

The devices of the present invention can be used for PUVA therapy. PUVA therapy is derived from the therapeutic application of psoralen (7H-furo[3,2-g]chromen-7-one) and derivatives thereof together with UV-A light. PUVA can be employed for the treatment of skin diseases characterized by hyperproliferative conditions. Psoralen is the parent compound in a family of natural products. It is structurally related to coumarines and can preferably be used for the treatment of psoriasis, eczema, vitiligo, mycosis fungoides, cuntaneous T-cell lymphoma, and other autoimmune diseases. With PUVA can also bet treated atopic eczema, lichen planus, urticaria pigmentosa, polymorphous light eruption, and alopecia areata.

Psoralen can be administered orally or topically to the skin. Preferred compounds are psoralen, 8-methoxypsoralen (8-MOP), 5-methoxypsoralen (5-MOP), and 4,5', 8-trimethylpsoralen (TMP). Afetr oral administration of 8-MOP, patients become gradually reactive to UV-A and therefore to photochemotherapeutic treatment. The patients are maximally reactive 2 to 3 hours after ingestion of the drug, and during this period the irradiation is carried out.

In the case of vitiligo khellin can be used instead of psoralen. The combined treatment with light and khellin is often called KUVA.

The devices of the present invention can also be used for photopheresis. Photophoreresis is a process by which peripheral blood is exposed in an extracorporeal flow system to photoactivate 5-MOP and represents a treatment for disorders caused by aberrant T lymphocytes. It is a therapy for advanced cutaneous T-cell lymphoma, pemphigus vulgaris and progressive systemic sclerosis (scleroderma). It can be used to treat autoimmune disorders. Further diseases that can be treated include multiple sclerosis, organ transplant rejection, rheumatoid arthritis, and AIDS.

The present invention particularly refers to devices according to the present invention for the use for the treatment of acneiform eruptions. The term acneiform eruption refers to a group of dermatoses including acne vulgaris, rosacea, folliculitis, and perioral dermatitis. Acneiform eruptions are, generally spoken, caused by changes in the pilosebaceous unit and are selected from acne aestivalis (Mallorca acne), acne conglobata, acne cosmetica, acne fulminans (acute febrile ulcerative acne), acne keloidalis (acne keloidalis nuchae, dermatitis papillaris capillitii, folliculitis keloidalis, folliculitis keloidis nuchae, nuchal keloid acne), acne mecanica, acne medicamentosa, acne miliaris necrotica (acne varioliformis), acne vulgaris, acne with facial edema (solid facial edema), acneiform eruptions, blepharophyma, erythrotelangiectatic rosacea (erthemaotelangiectatic rosacea), excoriated acne (acne excoriée des jeunes filles, Picker's acne), glandular rosacea, gnathophyma, gram-negative rosacea, granulomatous facial dermatitis, granulomatous perioral dermatitis, halogen acne, hidradenitis suppurativa (acne inversa, Verneuil's disease), idiopathic facial aseptic granuloma, infantile acne, lupoid rosacea (granulomatous rosacea, micropapular tuberculid, rosacea-like tuberculid of Lewandowsky), lupus miliaris disseminatus faciei, metophyma, neonatal acne (acne infantum, acne neonatorum), occupational acne, ophthalmic rosacea (ocular rosacea, ophthalmorosacea), otophyma, persistent edema of rosacea (chronic upper facial erythematous edema, Morbihan's disease, Rosaceous lymphedema), pomade acne, papulopustular rosacea, perifolliculitis capitis abscedens et suffodiens (dissecting cellulitis of the scalp, dissecting folliculitis, perifolliculitis capitis abscedens et suffodiens of Hoffman), perioral dermatitis, periorbital dermatitis (periocular dermatitis), pyoderma faciale (rosacea fulminans), rhinophyma, rosacea (acne rosacea), rosacea conglobata, rosacea fulminans, SAPHO syndrome, steroid rosacea, tropical acne.

Acne vulgaris (commonly called acne) is a common skin condition, caused by changes in pilosebaceous units, skin structures consisting of a hair follicle and its associated sebaceous gland, via androgen stimulation. It is characterized by noninflammatory follicular papules or comedones and by inflammatory papules, pustules, and nodules in its more severe forms. Acne vulgaris affects the areas of skin with the densest population of sebaceous follicles; these areas include the face, the upper part of the chest, and the back. Severe acne is inflammatory, but acne can also manifest in noninflammatory forms. Acne lesions are commonly referred to as pimples, blemishes, spots, zits, or simply acne.

Acne occurs most commonly during adolescence, affecting more than 89% of teenagers, and frequently continues into adulthood. In adolescence, acne is usually caused by an increase in male sex hormones, which people of both genders accrue during puberty. For most people, acne diminishes over time and tends to disappear - or at the very least decrease - after one reaches one's early twenties. There is, however, no way to predict how long it will take to disappear entirely, and some individuals will carry this condition well into their thirties, forties and beyond.

The face and upper neck are the most commonly affected, but the chest, back and shoulders may have acne as well. The upper arms can also have acne, but lesions found there are often keratosis pilaris. Typical acne lesions are comedones, inflammatory papules, pustules and nodules. Some of the large nodules are also called cysts and the term nodulocystic has been used to describe severe cases of inflammatory acne.

Aside from scarring, its main effects are psychological, such as reduced self-esteem and, in some cases, depression or suicide. Acne usually appears during adolescence, when people already tend to be most socially insecure. Early and aggressive treatment is therefore advocated by some to lessen the overall impact to individuals.

Light exposure can be used as treatment for acne. Used twice weekly, this has been shown to reduce the number of acne lesions by about 64% and is even more effective when applied daily. The mechanism appears to be that a porphyrin (Coproporphyrin III) produced within P. acnes generates free radicals when irradiated by 420 nm and shorter wavelengths of light. Particularly when applied over several days, these free radicals ultimately kill the bacteria. Since porphyrins are not otherwise present in skin, and no UV light is employed, it appears to be safe.

The treatment apparently works even better if used with a mixture of the violet/blue light and red visible light (e.g. 660 nm) resulting in a 76% reduction of lesions after three months of daily treatment for 80% of the patients; and overall clearance was similar or better than benzoyl peroxide. Unlike most of the other treatments few if any negative side effects are typically experienced, and the development of bacterial resistance to the treatment seems very unlikely. After treatment, clearance can be longer lived than is typical with topical or oral antibiotic treatments; several months is not uncommon. In addition, basic science and clinical work by dermatologists has produced evidence that intense blue/violet light (405 to 425 nm) can decrease the number of inflammatory acne lesion by 60 to 70% in four weeks of therapy, particularly when the P. acnes is pre-treated with delta-aminolevulinic acid (ALA), which increases the production of porphyrins.

The present invention therefore also relates to a combination of the said devices and active drugs for the treatment of therapeutic diseases. In particular, the present invention relates to the combined use of said devices and drugs used for the treatment of acne. The drugs can be selected from any drugs typically employed in order to treat acne, such as antibiotics (topical and/or oral), hormonal treatments, topical retinoids, topical bactericidals, sulfur. Suitable topical bactericidals are, for example, benzoyl peroxide, triclosan, and chlorhexidine gluconate. Suitable topical antibiotics are, for example, erythromycin, clindamycin, and tetracycline. Suitable oral antibiotics are, for example, erythromycin, tetracycline antibiotics (e.g. oxytetracycline, doxycycline, minocycline, or lymecycline), trimethoprim, and minocycline.

Suitable hormones are, e.g., selected from estrogen, progestogen, a combination of estrogen and progestogen, cyproterone, oestrogen, a combination of cyproterone and oestrogen, drospirenone, spironolactone, and cortisone. Suitable oral retinoids are, for example, vitamin A derivatives such as isotretinoin (e.g. Accutane, Amnesteem, Sotret, Claravis, Clarus). Suitable topical retinoids are, for example, tretinoin (e.g. Retin-A), adapalene (e.g. Differin), tazarotene (e.g. Tazorac), isotretinoin, and retinol. Further suitable drugs are , e.g. selected from anti-inflammatory drugs.

The devices according to the present invention can also be used in combination with dermabrasion to treat or prevent acne. Dermabrasion is a cosmetic medicinal procedure in which the surface of the skin is removed by abrasion (sanding).

Hereby any therapeutic strategy is included. The drug, e.g., can be administered first for a specific time period followed by the application of phototherapy using the devices according to the present invention. The time gap between both treatments may also vary, depending on the drug, its photoreactivity, individual circumstances of the subject, and the specific disease or condition. Both treatments may also overlap timely either partly or completely. The exact treatment strategy will depend on the individual circumstances and the severity of the disease or condition.

The combination therapy can have a synergistic effect and can reduce the side effects of traditional treatment strategies (e.g. the side effects of tetracyclines). This is due to the fact, that smaller doses of the drugs may be required when following the combined approach as outlined herein.

Comedones, also called blackhead, can also be treated by phototherapy employing the devices according to the present invention. A comedon is a yellow or blackish bump or plug on the skin. Actually, it is a type of acne vulgaris. Comedones are caused by excess oils that have accumulated in the sebaceous gland's duct. The substance found in these bumps mostly consists of keratin and modified sebum, which darkens as it oxidizes. Clogged hair follicles, where blackheads often occur, reflect light irregularly to produce a comedon. For this reason, the blockage might not necessarily look black when extracted from the pore, but may have a more yellow-brown colour as a result of its melanin content.

In contrast, a so called whitehead, which is also called closed comedo, is a follicle that is filled with the same material, sebum, but has a microscopic opening to the skin surface. Since the air cannot reach the follicle, the material is not oxidized, and remains white.

The device according to the present invention used for the treatment of acne preferably comprises at least one organic electroluminescent compound which emits light in the range between 350 and 900 nm, preferably between 380 and 850 nm, particularly preferably between 400 and 850 nm, and very particularly preferably between 400 and 800 nm.

Further particularly preferred light for the treatment of acne is blue light. Preferred blue light has emission wavelengths for the treatment of acne are 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429 and 430 nm. For example 414 and 415 nm are particularly suitable in order to kill P. acnes bacteria and to help cure existing blemishes and to prevent further outbreaks.
Studies on the application of phototherapy to treat acne revealed that a combination of different wavelengths or ranges of wavelengths are particularly suitable to treat acne efficiently. Particularly preferred is therefore a combination of red light and blue light to treat acne. The said red light is preferably selected from the range between 590 to 750 nm, particularly preferably between 600 and 720 nm, and very particularly preferably between 620 and 700 nm. Two further preferred wavelengths for the treatment of acne are 633 and 660 nm. The blue light can be selected from the wavelengths as described above.

In the case of comedo a composition comprising organic light emitting compound(s) emitting light with a wavelength of 500 nm or light in the range between 500 and 700 nm is particularly preferred.

Cellulite describes a condition that is claimed to occur in most women, where the skin of the lower limbs, abdomen, and pelvic region becomes dimpled. The causes of cellulite are poorly understood and may involve changes in metabolism and physiology such as gender specific dimorphic skin architecture, alteration of connective tissue structure, vascular changes and inflammatory processes. A couple of therapies are applied to prevent or to treat cellulite. Heat and the increase of blood flow are two common techniques. Therefore light therapy is considered to be beneficial to individuals suffering from cellulite. Devices according to the present invention are suitable for the treatment and/or prophylaxis of cellulite.
PDT is also suitable for the treatment and/or prophylaxis of cellulite.

The wavelength for the treatment and/or prophylaxis of cellulite that is to be emitted by the composition of the device according to the present inventionis in the range between 400 and 1000 nm, preferably in the range between 400 and 900 nm, particularly preferably between 450 and 900 nm, and very particularly preferably between 500 and 850 nm.

The more general term skin ageing refers to both the formation of wrinkles and hyperpigmentation. The signs of ageing of the human skin resulting from the effects on the skin of intrinsic and extrinsic factors are defined by the appearance of wrinkles and fine lines, by the yellowing of the skin which develops a wizened appearance along with the appearance of pigmentation blemishes, by a change in the thickness of the skin, generally resulting in a thickening of the stratum corneum and of the epidermis and a thinning of the dermis, by disorganization of the elastin and collagen fibers which causes a loss of elasticity, of suppleness and of firmness, and by the appearance of telnagiectasia.

Some of these signs are more particularly associated with intrinsic or physiological ageing, that is so to say with "normal" ageing associated with age, whereas others are more specific to extrinsic ageing, that is so to say ageing caused by the environment in general; such ageing is more particularly photo-ageing due to exposure to the sun. Other factors causing ageing of the skin are atmospheric pollution, wounds, infections, traumatisms, anoxia, cigarette smoke, hormonal status, neuropeptides, electromagnetic fields, gravity, lifestyle (e.g. excessive consumption of alcohol), repetitive facial expressions, sleeping positions, and psychological stressors.

The changes in the skin which occur due to intrinsic ageing are the consequence of a genetically programmed sequence involving endogenous factors. This intrinsic ageing in particular causes slowing down of the regeneration of skin cells, which is reflected essentially in the appearance of clinical damage such as a reduction of the subcutaneous adipose tissue and the appearance of fine lines or small wrinkles, and in histopathological changes such as an increase in the number and thickness of the elastic fibers, a loss of vertical fibers from the elastic tissue membrane and the presence of large irregular fibroblasts in the cells of this elastic tissue.

In contrast, extrinsic ageing results in clinical damage such as thick wrinkles and the formation of flabby and weather-beaten skin, and in histopathological changes such as an excessive accumulation of elastic substance in the upper dermis and degeneration of the collagen fibers.

There are different biological and molecular mechanisms which are responsible for he ageing of the skin and the process is currently not fully understood. However, it was recognized that both intrinsic and extrinsic factors of ageing of the skin share common mechanisms [P. U. Giacomoni et al., Biogerontology 2004, 2, 219-229]. These factors trigger a process leading to the accumulation of damages in the skin resulting in skin ageing since the expression of cell adhesion molecules provokes recruitment and diapedesis of circulating immune cells, which digest the extracellular matrix (ECM) by secreting collagenases, myeloperoxidases and reactive oxygen species.

The activation of these lytic processes provokes random damage of these resident cells, which in turn secrete prostaglandins and leukotrienes. These signaling molecules induce the degranulation of resident mast cells which release the autacoid histamine and the cytokine TNFalpha thus activating endothelial cells lining adjacent capillaries which release P-selectin and the synthesis of cell adhesion molecules such as E-selectin and ICAM-1. This closes a self-maintained micro-inflammatory cycle, which results in the accumulation of ECM damage, i.e. skin ageing.

There is a strong cosmetic and therapeutic need for novel strategies and compositions for the treatment or prophylaxis of skin ageing. Various cosmetic and therapeutic compositions (including for skin care) intended inter alia to prevent or treat ageing of the skin are known. Retinoic acid and derivatives thereof have been described as anti-ageing agents in skin care, cosmetic, or dermatological compositions, in particular in US 4603146. Hydroxy acids such as lactic acid, glycolic or alternatively citric acid are also known for this same application, these acids having been described in numerous patents and publications (e.g. EP-A-413528) and introduced into numerous skin care, cosmetic, or dermatological compositions on the market. Aromatic orthohydroxy acids such as salicylic acid have also been proposed (e.g. WO 93/10756 and WO 93/10755).

All of these compounds act against ageing of the skin by desquamation, that is to say removal of the dead cells at the surface of the stratum corneum. This desquamation is also referred as to a keratolytic property. However, these compounds also have side effects, consisting of stinging and redness, which the user finds unpleasant. Thus, there remains a need for anti-ageing agents which are at least as effective as the known compounds, but do not exhibit their drawbacks. Unlike the established strategies to treat or prevent skin ageing, modulating the selectin function is a novel concept intervening the micro-inflammation cascade at a very early stage and treating and preventing intrinsic and extrinsic skin ageing according to the present inventions represents a strategy without the drawbacks known from other strategies.

Phototherapy provides a new way to treat ageing of the skin. Thus, another subject of the invention is the use of the composition according to the present invention for the treatment and/or prophylaxis of skin ageing. This means, that the present invention provides solutions, inter alia, for skin rejuvenation and to reduce or prevent the formation of wrinkles.

The wavelength for the treatment of skin ageing that is to be emitted by the devices according to the present invention is in the range between 400 and 950 nm. Preferably the wavelength is in the range between 550 and 900nm, and particularly preferably between 550 and 860 nm.

The compositions of the devices of the present invention may also emit light of different wavelengths or wavelength ranges which also applies for other embodiments of the present invention.

The devices of the present invention can be used for the treatment of skin ageing emits light in the range of 600 nm and 650 nm, particularly preferably in the range between 620 nm and 650 nm.

The device according to the present invention can be used for the treatment and/or prevention of skin ageing. In this case the composition of the device preferably comprises at least one organic electroluminescent compound which emits light in the range between 350 and 950 nm, preferably between 380 and 900 nm, and particularly preferably between 400 and 900 nm.

Further particularly preferred light for the treatment and/or prophylaxis of skin ageing is blue light. Preferred blue light has emission wavelengths for the treatment and/or prophylaxis of skin ageing are 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, and 430 nm. For example 415 nm is particularly suitable.

Further particular preferred light for the treatment and/or prophylaxis of skin ageing has a wavelength between 400 and 900 nm.

Skin rejuvenation can also be achieved with light of the wavelength of 830 nm or slightly below or above that value. Therefore, compositions of the devices according to the present invention can also emit light in the range between 700 nm and 1000 nm, preferably between 750 nm and 900 nm, particularly preferably between 750 nm and 860 nm, and very particularly preferably between 800 nm and 850 nm.

Redness of the skin of a subject can be treated by a device according to the present invention. The wavelength for the treatment and/or prophylaxis of redness that is to be emitted by the composition of the device according to the present invention is in the range between 460 and 660 nm. Preferably the wavelength is in the range between 500 and 620 nm, and particularly preferably between 540 and 580 nm. One particular preferred wavelength for this purpose is 560 nm.

Dermatitis of a subject can be treated by a device according to the present invention. The wavelength for the treatment and/or prophylaxis of dermatitis that is to be emitted by the composition of the device according to the present invention is in the range between 470 and 670 nm. Preferably the wavelength is in the range between 490 and 650nm, and particularly preferably between 530 and 610 nm. Two particular preferred wavelengths for this purpose are 550 nm and 590 nm.

Atopic eczema of a subject can be treated by a device according to the present invention. The wavelength for the treatment and/or prophylaxis of atopic eczema that is to be emitted by the composition of the device according to the present invention is in the range between 470 and 670 nm. Preferably the wavelength is in the range between 490 and 650nm, and particularly preferably between 530 and 610 nm. One particular preferred wavelength for this purpose is 320 nm.

Psoriasis can be treated by a device according to the present invention. The wavelength for the treatment and/or prophylaxis of psoriasis that is to be emitted by the composition of the device according to the present invention is in the range between 240 and 500 nm. Preferably the wavelength is in the range between 290 and 400nm, and particularly preferably between 300 and 330 nm. Two particular preferred wavelengths for this purpose are 311 and 320 nm.

Vitiligo can be treated by a device according to the present invention. The wavelength for the treatment and/or prophylaxis of vitiligo that is to be emitted by the composition of the device according to the present invention is in the range between 240 and 500 nm. Preferably the wavelength is in the range between 290 and 400nm, and particularly preferably between 300 and 330 nm. One particular preferred wavelength for this purpose is 311 nm.

Targeted phototherapy has enabled therapeutic dosing of ultraviolet light to specific dermatoses while minimizing exposure of healthy skin. Specifically, the 308 nm wavelength of light within the ultraviolet B range has been shown as particularly effective for many dermatoses, including vitiligo; psoriasis; and leukoderma such as that associated with scars, striae alba and post-CO₂ laser resurfacing.

The devices of the present invention can also be used for the treatment of edema. Edema , formerly known as dropsy or hydropsy, is an abnormal accumulation of fluid beneath the skin or in one or more cavities of the body. Generally, the amount of interstitial fluid is determined by the balance of fluid homeostasis, and increased secretion of fluid into the interstitium or impaired removal of this fluid may cause edema. Five factors can contribute to the formation of edema: (1) It may be facilitated by increased hydrostatic pressure or by reduced oncotic pressure within blood vessels or (2) by increased blood vessel wall permeability as in inflammation or (4) by obstruction of fluid clearance via the lymphatic or (5) by changes in the water retaining properties of the tissues themselves. Raised hydrostatic pressure often reflects retention of water and sodium by the kidney.

The composition of the device according to the present invention used for the treatment of edema preferably comprises at least one organic electroluminescent compound which emits light in the range between 760 and 940 nm, preferably between 780 and 920 µm, particularly preferably between 800 and 900 µm, and very particularly preferably between 820 and 880 nm.

One further particularly preferred emission wavelength for the treatment of edema is 850 nm.

Another subject of the present invention relates to said device for the use in the treatment and/or prophylaxis of infections and inflammatory, neurological, and psychological diseases and/or conditions.

Many inflammatory diseases, disorder, and conditions can be treated with phototherapy. A device according to the present invention for the treatment and/or prophylaxis of inflammatory disorders is also subject of the present invention. Inflammatory diseases and conditions cover a wide range of indications. Many diseases or condition which are seemingly unrelated to inflammation have inflammatory components that can be treated with the compositions according to the present invention. The skin diseases and conditions mentioned in the present invention all have inflammatory components, such as acne, psoriasis, atopic dermatitis, eczema. A non limiting selection of further inflammatory diseases and conditions that can be treated with a device according to the invention are arthritis, inflammatory bowel disease, gingival inflammation, inflammation of the mucosa, inflammation of the nail bed, arteriosclerosis, and inflammation of the vascular system.

Preferred wavelengths for the treatment and/or prophylaxis of inflammation are in the range between 350 and 900 nm, particularly preferably between 380 and 900 nm, and very particularly preferably between 400 and 860 nm. Further preferred wavelengths for the treatment and/or prophylaxis of inflammation are 405, 420, and 850 nm.

The said devices can be used for the treatment and/or prophylaxis of infections. Infections can be caused by bacteria and viruses. Light has several positive effects on infections. Light has, e.g., anti-inflammatory effects through the stimulation of the tissue as outlined elsewhere within the present invention.

Phototherapy with devices according to the present invention are beneficial for the use to treat wounds. Wound healing is often associated with inflammation. Therefore the same wavelengths and ranges of wavelengths as outlined for the treatment and/or prophylaxis of inflammation can be applied. Treating wounds by phototherapy also prevents the formation of scares. Particularly preferred wavelengths for the treatment and/or prophylaxis of wounds and/or scares are in the range between 600 and 950 nm and very particularly preferably between 650 and 900 nm. Further preferred wavelengths for the treatment and/or prophylaxis of wounds and scares are 660, 720, and 880 nm.

Other infections that can efficiently be treated with devices according to the present invention are caused by bacteria.

Further infections that can efficiently be treated with devices according to the present invention are caused by viruses. A preferred embodiment of this invention is the use of the said OLECs comprising for the treatment and/or prophylaxis of viral infections particularly caused by cytomegalovirus (CMV), encephalo myocarditis virus (EMCV), poliovirus, influenca virus, parainfluenza respiratory influenza virus, respiratory syncytial virus, Japanese encephalitis virus, Dengue virus, hepatitis A virus (HAV), hepatitis B virus (HBV), hepatitis C virus (HCV), hepatitis. D virus (HDV), hepatitis E virus (HEV), hepatitis F virus (HFV), hepatitis G virus (HGV) Epstein Barr Virus (EBV), human immunodeficiency virus type 1 (HIV-I), human immunodeficiency virus type 2 (HIV-2), varicella zoster virus, herpes simplex virus, in particular herpes simplex virus type 1 (HSV-I), herpes simplex virus type 2 (HSV-2), or human herpes virus 1, 2, 3, 4, 7, or 8, Kaposi's sarcoma-associated herpesvirus (KSHV), rotavirus, papilloma virus, and human papilloma virus (HPV), in particular HPV of the types: 1, 2, 3, 4, 5, 8, 9, 11, 12, 13, 14, 15, 16, 17, 18, 19-29, 31, 32, 34, 36-38, 46-50, 56, or 58.

In particular viral skin diseases and/or tumor disorders can be treated with the devices according to the present invention such as genital warts, benign tumors of the skin and/or mucosa caused by papilloma viruses, in particular verrucae plantares, verrucae vulgares, verrucae planae juveniles, epidermodysplasia verruciformis, Condylomata acuminate, Condylomata plana, bowenoid papulosis, papilloma on the larynx and oral mucosa, focal epithelial hyperplasia, herpes labialis, varicella and shingles.

In a particularly preferred embodiment of the present invention the device of the invention can be used for the treatment and/or prophylaxis of warts. Pulsed light therapy might be one way to treat warts with devices according to the present invention.

A device according to the present invention for the treatment and/or prophylaxis of neurological or psychological diseases and/or conditions is also subject of the present invention.

A preferred neurological disease according to the present invention is Morbus Parkinson (MB). When light reaches a certain level of intensity, it inhibits melatonin which in turn limits the production of dopamine. By limiting the melatonin is supposed to lead to a have better production and use of dopamine in the brain. Recent case studies of light therapy on MB patients involving bright light therapy have had positive results with marked improvement in bradykinesia and rigidity in most patients while being exposed for only ninety minutes.

Further preferred neurological and psychological diseases and/or conditions according to the present invention are mood and sleep related. Light is well known to be beneficial on the mood in many circumstances. Phototherapy can also be employed to treat depression, seasonal affective disorder (SAD), non seasonal depression, circadian rhythm sleep disorder (chronic circadian rhythm sleep disorder (CRSD), situational CRSD).

The US National Library of Medicine notes that some people experience a serious mood change when the seasons change. They may sleep too much, have little energy, and crave sweets and starchy foods. They may also feel depressed. Though symptoms can be severe, they usually clear up. The condition in the summer is often referred to as Reverse Seasonal Affective Disorder, and can also include heightened anxiety. It has been estimated that 1.5 to 9% of adults in the US experience SAD.

There are different treatments for classic (winter-based) seasonal affective disorder, including light therapy with bright lights, antidepressant medication, cognitive-behavioral therapy, ionized-air administration, and carefully timed supplementation of the hormone melatonin.

The wavelength for the treatment and/or prophylaxis of these neurological and psychological diseases and/or conditions that is to be emitted by the composition of the device according to the present invention is in the range between 350 and 600 nm. Preferably the wavelength is in the range between 400 and 550nm, and particularly preferably between 440 and 500 nm. Two particular preferred wavelengths for this purpose are 460 and 480 nm.

The devices according to the present invention may also be used for the treatment and/or prophylaxis of pain. Pain relief by phototherapy is well known. The following conditions produce pain that can be treated successfully with phototherapy: carpal tunnel syndrome, chronic wounds, epicondylitis, headache, migraine, plantar fasciitis, tendonditis and bursitis, neck pain, back pain, muscle pain, trigeminal neuralgia, and Whiplash-associated injuries.

Preferably, muscle pain is treated with devices emitting red or infrared light.

Alopecia areata is a condition affecting humans, in which hair is lost from some or all areas of the body, usually from the scalp. Because it causes bald spots on the scalp, especially in the first stages, it is sometimes called spot baldness. In 1 to 2% of cases, the condition can spread to the entire scalp (alopecia totalis) or to the entire epidermis (alopecia universalis). Conditions resembling alopecia areata, and having a similar cause, occur also in other species.

Alopecia areata (autoimmune hair loss) can be treated with a device according to the present invention. The wavelength for the treatment and/or prophylaxis of alopecia areata that is to be emitted by the composition of the device according to the present invention is in the range between 240 and 500 nm. Preferably the wavelength is in the range between 290 and 400 nm, and particularly preferably between 300 and 330 nm. One particular preferred wavelength for this purpose is 311 nm.

A device according to the present invention can also be used for the disinfection of beverages and nutrition.

The use of light as disinfectant is well known. The devices according to the present invention can be used for disinfection. Hereby any kind of disinfection is meant and includes without limitation the disinfection of wounds, nutrition, and solid and liquids objects, such cosmetic, medical devices, devices used for surgery and beverages.

The devices can be used for the disinfection of beverages, preferably water, and particularly preferably drinking water. Contaminated water causes many infections worldwide and leads often to severe diseases or death of the individuals.
Water filter systems of commercial providers (e.g. BRITA water filter system) take advantage of ion exchange technology. The filter, however, tend to microbial contamination, which, in turn results in water which is contaminated with microbes. One solution is to add silver salt which is from a toxicological point of view problematic. The devices of the present invention provide a solution to this problem. They can be incorporated into the water filter system in order to provide a safe, efficient, and low cost way to provide water with a low degree of microbial contamination. The light source can irradiate both the water before or after filtering or the filter cartridge itself. Preferably the light source comprising the devices irradiates both the filter cartridge and the already filtered water.

The procedure of disinfection of water as outlined above can basically be applied to any other liquid, in particular beverage analogously.

Therefore, the devices according to the present invention can be used for the disinfection of beverages and nutrition for humans and animals.

Wavelengths for disinfection according to the present invention are in the range between 200 nm and 600 nm, preferably between 250 nm and 500 nm, and very particularly preferably between 280 nm and 450 nm.

In another embodiment the present invention relates to the said devices for the application in photodynamic therapy (PDT).

Wavelengths required for PDT according to the present invention are in the range between 300 and 700 nm, preferably between 400 and 700 nm, and very particularly preferably between 500 and 700 nm. Four further preferred wavelengths are 595, 600, 630, and 660 nm.

Any therapy known as PDT can be treated with devices according to the present invention. In particular PDT as outlined within the present invention can be treated with devices according to the present invention. The property of dyes with a polycyclic hydrocarbon type chemical structure to accumulate in greater amounts in tumor tissues than in normal tissues is well known. The dyes include acridines, xanthenes, psoralens, and porphyrins. The latter dyes, in particular, hematoporphyrin (Hp) and some of its chemical derivatives (e.g. Hp D, wherein Hp D is a mixture of Hp derivatives), have superior tumor-localizing properties, which are the basis of phototherapeutic treatment of tumors with red light irradiation at predetermined times after systemic administration of the drug..

Drugs used for PDT are preferably selected from aminolevulinic acid/methyl aminolevulinate, efaproxiral porphyrin derivatives (porfimer sodium, talaporfin, temoporfin, verteporfin).

In a further embodiment the present invention relates to the said devices for the treatment and/or prophylaxis of jaundice and crigler naijar, preferably jaundice.

Jaundice, which is also known as icterus, is a yellowish discoloration of the skin, the conjunctival membranes over the sclerae (whites of the eyes), and other mucous membranes. The discoloration is caused by hyperbilirubinemia (increased levels of bilirubin in the blood). This hyperbilirubinemia subsequently causes increased levels of bilirubin in the extracellular fluids. Jaundice is classified in three groups, pre-hepatic (hemolytic) jaundice, hepatic (hepatocellular) jaundice, and post-hepatic (obstructive) jaundice.

Pre-hepatic jaundice is caused by anything which causes an increased rate of hemolysis, i.e. breakdown of red blood cells. In tropical countries, malaria can cause jaundice in this manner Certain genetic diseases, such as sickle cell anemia, spherocytosis and glucose 6-phosphate dehydrogenase deficiency can lead to increased red cell lysis and therefore hemolytic jaundice. Commonly, diseases of the kidney, such as hemolytic uremic syndrome, can also lead to coloration. Defects in bilirubin metabolism also present as jaundice. Jaundice usually comes with high fevers. Rat fever (leptospirosis) can also cause jaundice.

Hepatic jaundice causes include acute hepatitis, hepatotoxicity and alcoholic liver disease, whereby cell necrosis reduces the liver's ability to metabolise and excrete bilirubin leading to a buildup in the blood. Less common causes include primary biliary cirrhosis, Gilbert's syndrome (a genetic disorder of bilirubin metabolism which can result in mild jaundice, which is found in about 5% of the population), Crigler-Najjar syndrome, metastatic carcinoma and Niemann-Pick disease, type C. Jaundice seen in the newborn, known as neonatal jaundice, is common, occurring in almost every newborn as hepatic machinery for the conjugation and excretion of bilirubin does not fully mature until approximately two weeks of age.

Post-hepatic jaundice, also called obstructive jaundice, is caused by an interruption to the drainage of bile in the biliary system. The most common causes are gallstones in the common bile duct, and pancreatic cancer in the head of the pancreas. Also, a group of parasites known as "liver flukes" can live in the common bile duct, causing obstructive jaundice. Other causes include strictures of the common bile duct, biliary atresia, ductal carcinoma, pancreatitis and pancreatic pseudocysts. A rare cause of obstructive jaundice is Mirizzi's syndrome.

Jaundice, in particular neonatal jaundice, can lead to severe medical consequences if not or not appropriately treated. Increased concentrations of bilirubin can result in a brain-damaging condition known as kernicterus, leading to significant lifelong disability; there are concerns that this condition has been rising in recent years due to inadequate detection and treatment of neonatal hyperbilirubinemia. Early treatment often consists of exposing the infant to intensive phototherapy in an more or less isolated incubator. The therapy often represents an emotionally or psychologically difficult situation for both the infant and the parents. The compositions of the present invention can be employed in order to provide flexible and ambulatory devices such as blankets. Thus, the infant can be treated while laying in its parents' arms. Traditional therapies also easily lead to overheating of the infant, which can also be significantly reduced with the devices of the present invention.

Preferably the present invention relates to devices used for the treatment of neonatal jaundice.

Jaundice of a subject can be treated by a device according to the present invention. The wavelength for the treatment and/or prophylaxis of jaundice that is to be emitted by the composition of the device according to the present invention is in the range between 300 and 700 nm. Preferably the wavelength is in the range between 350 and 600nm, and particularly preferably between 370 and 580 nm. Further preferred wavelengths are in the range between 400 and 550 nm. Particularly preferred wavelengths are in the range between 410 and 470 nm. Two particular preferred wavelengths for this purpose are 450 and 466 nm.

The devices according to the present invention can also be used for cosmetic applications.

For operating, the compositions of the present invention need to be incorporated into a device.
Both the composition and the therapeutic and cosmetic conditions have already been described in detail above. The device can have any shape, be rigid or flexible. The device requires energy supply in any form. The energy supply may be directly associated to the device or separated by, e.g., a cable. According to claim 1 a printable battery is attached to the device in order to provide a device which is comfortable for the subject to be treated forming a totally self-contained portable unit. Irradiation may, thus, occur at any time and at any place without disturbing the subject to be treated in its habits or daily life. Home use of devices according to the present invention is particularly preferable. The device may be self adhesive and detachable. It may conform a planar or non-planar portion of the body or be an implantable probe.

The device may comprise an interactive steering unit. The steering unit may allow a switch from continuous illumination to pulsed illumination. It also may allow the precise adaptation of irradiation intensities and/or wavelengths to be emitted. The steering unit may be directly associated to the device. It can also be separated via a permanent or temporary linkage. The device may be disposable and is suitable for uses in the hospital or outside the hospital.

In any case the device according to the present invention is suitable as light weight device for portable use. However, stationary devices can also be prepared. The device is sufficiently portable to enable ambulatory treatment i.e. treatment in which the subject can move around freely. It can be subsequently removed in the human subject's own time, so that treatment could take place almost everywhere. This results in a better convenience and lower costs (from avoiding either an out-patient or inpatient stay in hospital).

In the case of PDT the treatment is often associated with pain. Ambulatory devices according to the present invention can be used with lower light levels since exposure can occur for a longer period of time. This overcomes a problem of pain induced in some patients by the high irradiances from conventional sources used in hospitals. In addition lower irradiance is more effective in PDT due to reduction of the extent of photobleaching of the photopharmaceutical.

The devices may be provided with a photochemical and/or a photopharmaceutical preparation present. This may be in the form of a gel, ointment or cream. Alternatively, or as well, the device may be provided with a thin film impregnated with the photopharmaceutical. Typically, the photopharmaceutical preparation is provided as a layer in contact with the light source. Provided that the photopharmaceutical preparation is transparent or sufficiently translucent for the frequency of stimulating light, the resulting device can be readily applied without a separate step of applying the photopharmaceutical to a patient. Creams which would scatter the light may nevertheless be used if they are absorbed before the light source is switched on. A photopharmaceutical layer may be covered by a peelable release medium, such as a silicone-backed sheet. The photopharmaceutical preparation may comprise an inactive compound which is metabolised in vivo to an active compound. Delivery of the photopharmaceutical can be assisted by iontophoresis.

The output of light from the organic light-emitting semiconductor may be pulsed and an electronic control circuit or microprocessor may be provided to control this pulsing and/or other aspects of device function such as duration of exposure(s) of the area to be treated and the intensity of emitted light. Pulsed devices may be provided with a preparation of a photochemical and/or a photopharmaceutical substance which is photobleachable or which is metabolised in vivo to a photobleachable chemical species.
The output of the device may take the form of a train of pulses, preferably in which the duration of the pulses is substantially the same as the interval between successive pulses. The period of the pulse train may, for example, be in the range of 20 ms to 2000 s, depending on the photobleaching characteristics of said substance.
Preferably, the attachment means comprises an adhesive surface to enable the device to be attached to a patient.
Further preferred features correspond to the first aspect above.

Preferably, the ambulatory device is provided with a photochemical and/or a photopharmaceutical preparation present. Preferred features of the preparation and its delivery are as above. In particular, the photochemical and/or photopharmaceutical may be photobleachable or may be metabolised in vivo to a photobleachable chemical species.
The means for activating and deactivating the source may control other aspects of device function such as duration of exposure(s) of the area to be treated and the intensity of emitted light.
The control means may to advantage be operable to cover the source to emit a pulse train having any one or more of the preferred features of the pulse train produced by a device in accordance with the first aspect of the invention.
Suitable devices according to the present invention are selected from sleeves, bandages, pads, plaster, implantable probes, nasogastric tubes, chest drains, stents, clothe like devices, blankets, sleeping bags, devices fitting one or more teeth in the mouth, and patches.

The device may be used as a stent, for example a tube of 1.25 to 2.25 cm radius of say 10 to 12 cm length for use inside the oesophagus.

The device may be a blanket or sleeping bag in order to treat, e.g., jaundice of infants. Currently infants suffering from jaundice are separated from their parents and illuminated in incubators blindfold. This represents an unpleasant situation for both the infant and the parents. In addition, the infant is not able to adjust his body temperature as easily as adults can do and overheating in the incubator is a critical issue. Flexible blankets and sleeping bag provide a way to treat the infant without these problems. The infant covered by the blanket or sleeping bag can be irradiated while laying in his parents' arms and overheating of the infant's body is not as critical as compared to traditional therapies. This is due to the fact that the devices according to the present invention require less power and produce, in turn, less heat.

In psoriatic patients plaques are often found in body folds. Conventional phototherapy represents a problem which is due to the fact that light emitted by a light source does not reach the plaque in the body folds. OLEDs theoretically offer the opportunity to design a light source with direct contact to the psoriatic skin in the body fold. As outlined above curved surfaces represent a technical difficulty when manufacturing OLEDs. The problem can, however, be solved with OLECs. OLECs can be designed to fit into body folds in order to treat psoriasis and other diseases and/or conditions found in body folds.

Devices can generally spoken individually tailored in any form that is required for treatment.

The device itself may comprise a therapeutic agent which is released in a controlled way during the treatment.
Preferably the said device comprise a plastic ionic material as described above, which has a glass transition temperature Tg or melting point in the range between 25 and 45°C. Thus, the device will getting softer when attached to the skin in order to get a better contact to the skin.

As outlined above, OLECs are particularly suited for the application in phototherapy and PDT. They are rather simple in terms of structure and manufacturing, which reduces production costs. More advantages of OLECs have already been discussed within the present invention. The OLECs preferably comprise at least two electrodes, particularly preferably two electrodes, a cathode and an anode. Both electrodes are connected through a composition that has been described above.

Preferred materials for the electrodes used in OLECs are selected from metals, particularly preferably selected from Al, Cu, Au, Ag, Mg, Fe, Co, Ni, Mn, Zn, Cr, V, Pd, Pt Ga, In and their alloys, conductive oxide, for example ITO, AZO, ZnO, and conductive organic thin films comprising such as poly(ethylenedioxythiophene)-polystyrene sulfonate (PEDOT:PSSH), Polyaniline (PANI). Further suitable conducting polymers could be found for example in the reviews edited by Michael S. Freund & Bhavana'Deore, in "Self-Doped Conducting Polymers", John Willey & Sons, Ltd., 2007.

Preferably, the OLECs are prepared on a flexible substrate. The suitable substrate is preferably selected from films or foils based on polymers or plastics. The main selection criteria for polymers or plastics are 1) hygienic property and 2) glass transition temperature. The glass temperature (Tg) of the polymers can be found in a common handbooks, e.g. in "Polymer Handbook", Eds. J. Brandrup, E. H. Immergut, and E. A. Grulke, John Willey & Sons, Inc., 1999, VI/193-VI/276. Preferably, the Tg of the polymer is above 100°C, particularly preferably above 150°C, and very particularly preferably above 180°C. Very preferred substrates are for example, poly(ethylene terephthalate) (PET) and poly(ethylene 2,6-naphthalate) (PEN).
To avoid degradations caused by oxygen and moisture, and also to prevent active materials in the devices, for example the ionic compounds and the organic electroluminescent compounds from being in contact with the subject to be treated, an appropriate encapsulation for the said device is a prerequisite for the applications in therapeutic treatments and cosmetic conditions.

There are many technologies suitable for encapsulation of the devices according to the present invent. In general, all encapsulation techniques, which are developed for organic light emitting diodes (OLEDs), organic solar cells, organic dye-sensitized solar cells, organic field-effect transistor (OFETs), thin film batteries, microelectromechanical systems (MEMS) and electronic papers, can be applied in order to encapsulate the devices according to the present invention.

In a preferred embodiment, the device of the present invention is encapsulated using a thin film encapsulation. Typically, a thin film encapsulation consists of a multi alternating layers of an inorganic/organic stack, wherein inorganic layers are used to achieve adequate barrier performance and organic layers to eliminate inevitable defects of the inorganic layers. The materials used for inorganic layers can be selected from metals, metal oxides or mixed oxides, for example Ag, SiOₓ, SiNx, AlOₓ, ZrOₓ, ZnOₓ, HfOₓ, TiOₓ and indium tin oxide and so on. Some examples are alternating multilayers of vacuum-deposited acrylate polymers/AlOₓ as reported by Graff, G. L. et al. (J. Appl. Phys. 2004, 96, 1840), Al₂O₃/polyurea layers as reported by Young Gu Lee et al. (Org. Electron. 2009, 10, 1352 and in Dig. Tech. Pap.-Soc. Inf. Disp. Int. Symp. 2008, 39, 2011), SiON/SiO₂/parylene on PET substrate as reported by Han,Jin Woo, et al. (Jpn. J. Appl. Phys., Part 1 2006, 45, 9203), and polyacrylate(20 µm)-Ag(200 nm) as reported by Wang,Li Duo et al. (Chin. Phys. Lett. 2005, 22, 2684).

By using advanced deposition techniques, for example atomic layer deposition (ALD), plasma assisted pulsed laser deposition (PAPLD) and plasma enhanced chemical vapor deposition (PECVD), the defects in inorganic layer can be significantly reduced so that all inorganic layers can be used, for example Al₂O₃/HfO₂ nanolaminated films by ALD as reported by Chang,Chih Yu et al. (Org. Electron. 2009, 10, 1300), and SiNx/ SiOx layers as reported by Li,C.Y. et al. (IEEE Electron. Compon. Technol. Conf. 2008, 58th, 1819), (PECVD SiO)/ poly-benzo-oxazole (PBO) by Shimooka,Y. et al.( IEEE Electron. Compon. Technol. Conf. 2008, 58th, 824), nanolaminated alternating layers of Al₂O₃/ZrO₂ by Meyer,J. et al. (Appl. Phys. Lett. 2009, 94, 233305/1), and nanolaminates of Al₂O₃/ZrO₂ by PAPLD as reported by Gorrn,Patrick et al. (J. Phys. Chem. 2009, 113, 11126), and SiC layers by PECVD as reported by Weidner,W.K. et al. (Annu. Tech. Conf. Proc - Soc. Vac. Coaters 2005, 48th, 158), multilayer stack of silicon nitride - silicon oxide - silicon nitride silicon oxide - silicon nitride (NONON) by PECVD as reported by Lifka,H., et al. (Dig. Tech. Pap.- Soc. lnf. Disp. Int. Symp. 2004, 35, 1384), and polyethersulfon (PES)/ ALD AlOₓ as reported by Park,Sang-Hee Ko, et al. (ETRI Journal 2005, 545). A review on thin film encapsulation by CVD and ALD is provided by Stoldt, Conrad R, et al.(J. Phys. D: Appl. Phys. 2006, 39, 163).

Further single layer encapsulation was also developed. Examples of single barrier layers are a perfluorinated polymer (Cytop), which can be easily spin-coated on OLEDs, as reported by Granstrom, J. et al. (Appl. Phys. Lett. 2008, 93, 193304/1), and single layer consisting of aluminum oxynitride (AlOₓN_{y}) by using a reactive radio frequency (RF) magnetron sputtering as reported by Huang, L.T. et al. (Thin Solif Films 2009, 517, 4207), single poly-SiGe layer by PECVD as reported by Rusu, Cristina et al. (J. Microelectromech. Syst. 2003, 12, 816).

Further details on materials and methods for encapsulation are disclosed, e.g., in WO 2009/089417, WO 2009/089417, WO 2009/042154,
WO 2009/042052, US 2009/081356, US 2009/079328,
WO 2008/140313, WO 2008/012460, EP 1868256, KR 2006/084743, KR 2005/023685, US 2005/179379, US 2005/023974, KR 2003/089749, US 2004/170927, US 2004/024105, WO 2003/070625, and
WO 2001/082390.

In another preferred embodiment, the device of the present invention is encapsulated by using a curable resin together with a cap, wherein the cap covers at least the light emitting area, and the curable resin is applied between the substrate and the cap. The cap materials can be selected from metals and plastics in form of a plate or foil, and glass cap. Preferably, the cap is flexible, which is preferably selected from metal foils, plastic foils or metallised plastic foils. The metal can be selected from Al, Cu, Fe, Ag, Au Ni, whereby Al is particularly preferred. The selection criteria for plastics are 1) hygienic aspects 2) the glass transition temperature (T_{g}), which is supposed to be high enough. Tg of polymers can be found in a suitable handbook, for example in "Polymer Handbook", Eds. J. Brandrup, E. H. Immergut, and E. A. Grulke, John Willey & Sons, Inc., 1999, VI/193-VI/276. Preferably, the polymer suitable for cap material has a T_{g} above 60°C, preferably above 70°C, particularly preferably above 100°C, and very particularly preferably above 120°C. The cap used in the present invention is poly(ethylene 2,6-naphthalate) (PEN).

The suitable resin can be thermally cured or UV-curable. Preferably, the resin is UV-curable, optionally supported or facilitated by heating. A typical resin is the epoxy-based resin, which is commercially available at for example Nagase & Co., LTD. and DELO Industrie Klebstoffe. The resin can be applied on full-area of the emitting area or just on the edge, where no light emitting area is underneath.

Preferred electrode materials can selected from all metals, preferably Al, Cu, Au, Ag, Mg, Fe, Co, Ni, Mn, Zn, Cr, V, Pd, Pt and their alloys, conductive oxide, for example ITO, AZO, ZnO etc., and conductive organic thin films comprising PEDOT:PSSH, PANi etc.

Preferably, the OLECs are prepared on a flexible substrate. The suitable substrate is preferably selected from films or foils based on polymers or plastics. The selection criterion for polymers or plastics are 1) hygienic property 2) glass transition temperature. The glass temperature (Tg) of the polymers can be found in a suitable handbook, for example in "Polymer Handbook", Eds. J. Brandrup, E. H. Immergut, and E. A. Grulke, John Willey & Sons, Inc., 1999, VI/193-VI/276. Preferably, the Tg of the polymer is above 100°C, very preferably above 150°C, and particularly above 180°C. Very preferred substrates are for example, poly(ethylene terephthalate) (PET) and poly(ethylene 2,6-naphthalate) (PEN)

In a preferred embodiment, the said composition further comprises an ion conductor, which is preferably selected from polymeric materials, such as perfluorosulfonic acid-based formulations, polybenzimidazoles, sulfonated polyetherketone, sulfonated naphthalenic polyimides, and polyethylene oxide (PEO)-based formulations. Further suitable polymers can be selected from the polymers for proton-exchange membrane for fuel cells. Such polymers are disclosed, for example, in the review by Hickner et al., "Alternative Polymer Systems for Proton Exchange Membranes (PEMs)" in Chemical Reviews, 2004, 104, 4587-4612. A very preferred ion conductor for the present invention is polyethylene oxide (PEO).

The device according to the present invention emits electromagnetic radiation to cause said treatment and/or prophylaxis of the area, wherein the OLEC has an extent of at least 0.5 cm². The OLEC can be continuous or discontinuous. The OLEC and its illuminating area can adopt any shape that is suitable for the treatment. This can, in particular in therapeutic conditions, prevent side effects through the irradiation of parts of the subject whose treatment is not required.

In a further preferred embodiment the device of the present invention has an extent between 0.5 cm² and 100000 cm², particularly preferably between 0.5 cm² and 50000 cm².

In a further preferred embodiment the device according to the present invention is an ambulatory device.

The present invention also relates to a device, characterized in that it comprises an attachment means for attaching the device to a patient.

The device can be self adhesive or can be temporarily fixed at the side of action with an auxiliary material such as a glue strip.

The said device is characterized in that it can be a plaster, bandage, blanket, sleeping bag, sleeve, implantable probe, nasogastric tube, chest drain, pad, stent, and patch. The form and shape of the device can be tailored according to the individual needs of the treatment and according to the constitution of the subject to be treated.

The device according to this invention comprises a power supply unit. As outlined above the power supply can be directly attached to the device. This allows the design of ultra-thin devices which, e.g., can be used under the clothes without disturbing the subject to be treated. The power supply can also be in a more separated unit which is connected to the device in any possible way in order to supply the power.

The device according to the present invention is intended to illuminate parts of the subject.

Other features of the invention will become apparent in the course of the following description of exemplary embodiments, which are given for illustration of the invention and are not intended to be limiting thereof. the treatment and/or prophylaxis therapeutic and/or cosmetic diseases and conditions in animals and humans.

Another subject of the present invention is the use of a composition or a device according to this invention for the treatment and/or prophylaxis of therapeutic diseases. The therapeutic diseases are the same as outlined above.

When a human subject is to be treated cosmetic applications have an important function. The use of a composition or a device according to the present invention for the treatment and/or prophylaxis of cosmetic conditions is also subject of the present invention. The cosmetic conditions are the same as outlined above.

Further, the present invention relates to a method for the treatment and/or prophylaxis of diseases of humans and/or animals by using the compositions according to the present invention.

The present invention also relates to a method for the treatment and/or prophylaxis of cosmetic conditions of humans and/or animals by using the compositions according to the present invention.

The said methods can be used to treat the skin and other parts of the human or animal body as outlined above.

In another embodiment the present invention relates to a method for the treatment and/or prophylaxis of diseases of humans and/or animals by using devices comprising the said compositions to irradiate parts of the body of humans and/or animals, preferably the skin.

In another embodiment the present invention relates to a method for the treatment and/or prophylaxis of cosmetic conditions of humans and/or animals by using devices comprising the said compositions to irradiate parts of the body of humans and/or animals, preferably the skin.

The used terms compositions, devices, (therapeutic) diseases, cosmetic conditions etc. are the same as defined elsewhere within the present application.

It will be appreciated that variations to the foregoing embodiments of the invention can be made while still falling within the scope of the invention. Each feature disclosed in this specification, unless stated otherwise, may be replaced by alternative features serving the same, equivalent or similar purpose. Thus, unless stated otherwise, each feature disclosed is one example only of a generic series of equivalent or similar features.

All of the features disclosed in this specification may be combined in any combination, except combinations where at least some of such features and/or steps are mutually exclusive. In particular, the preferred features of the invention are applicable to all aspects of the invention and may be used in any combination. Likewise, features described in non-essential combinations may be used separately (not in combination).

It will be appreciated that many of the features described above, particularly of the preferred embodiments, are inventive in their own right and not just as part of an embodiment of the present invention. Independent protection may be sought for these features in addition to or alternative to any invention presently claimed.

The teaching as disclosed here can be abstracted und combined with other examples disclosed.

Other features of the invention will become apparent in the course of the following description of exemplary embodiments, which are given for illustration of the invention and are not intended to be limiting thereof.

### Working Examples

### Example 1

### Materials

The following polymers IL1, BE1, and RE2 are synthesized by employing the Suzuki coupling. The reaction can be carried out according to synthetic methods well known to the person skilled in the art. The method is described, e.g., in WO 2003/048225.

Polymer IL1, used as interlayer, is the copolymer comprising the following monomers with mol% as indicated:

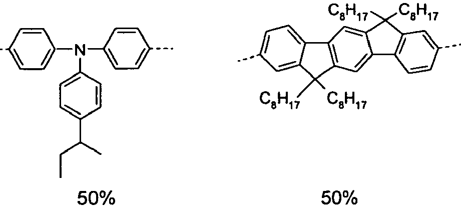

The molecular weight (MW) of the resulting polymer IL1 is distributed between 200000 to 300000 g/mol.

BE1, as shown bellow, is a copolymer of 50% phenanthrene and 50% spiro-bifluorene, which emits light in the deep blue region from 380 to 500 nm, with a maximum close to 420 nm.

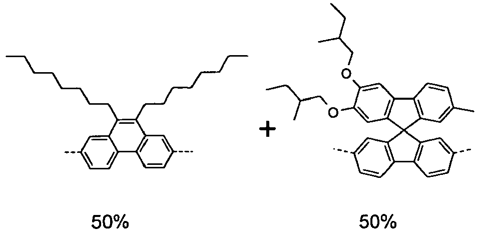

The molecular weight (MW) of the resulting polymer BE1 is distributed between 200000 to 300000 g/mol.

YE1 (Super Yellow, PDY-132, available from Merck KGaA, Germany) is a PPV (poly(para-phenylene vinylene)) polymer emitting yellow light with a broad emission between 500 to 700 nm. YE1 is are synthesized by employing the Gilch polymerization. The reaction can be carried out according to synthetic methods well known to the person skilled in the art. The method is described, e.g., in EP 1029019 B1.

RE1 is an ionic transition-metal complex Ru(bpy)₃²⁺(PF₆⁻)₂ as shown bellow with PF₆⁻ as counterion. RE1 has a broad emission from 560 to 800 nm with a peak around 630 nm. The synthesis of RE1 is as follows: Ru(bpy)₃Cl₂ is purchased from Alfa and is used as received. RE1 is obtained by counterion exchange to PF₆⁻ via a metathesis reaction in which RE1 is precipitated from aqueous solutions of the corresponding Cl⁻ salts and an excess of NH₄PF₆, washed with water, and dried.

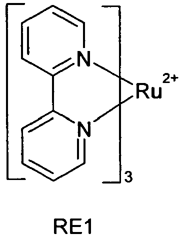

RE2 is conjugated copolymer containing Iridium metal complex on main chain having a molecular weight (MW) of about 300000 to 500000 g/mol.

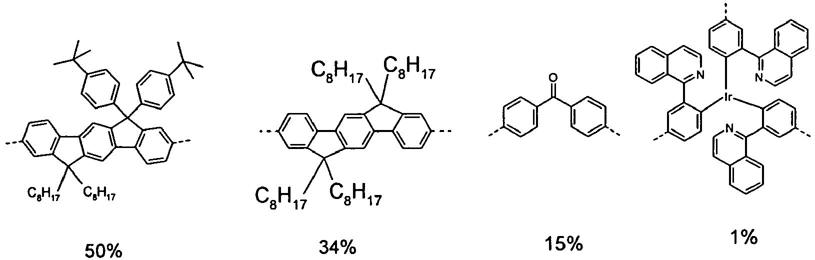

Poly(methyl methacrylate) (PMMA) is used as matrix for ionic transition-metal complex RE1.

Poly(ethylene oxide) (PEO) is used as ion conducting material. PEO having a molecular weight of MW = 5 x 10⁶ is purchased from Aldrich, and is used as received.

The salt KCF₃SO₃ (IM1) and tetrabutylammonium tetracyanoborate (IM2) are two ionic materials (IMs). IM1 is purchased from Alfa Aesar, and is used as received. The synthesis of IM2 can be referred to Z. Anorg. Allg. Chem. 2000, 626, 560-568.

### Example 2

### Formulations 1 to 4

In the following 4 different Formulations comprising compositions according to the present invention are prepared employing standard techniques known to the person skilled in the art.

### a) Preparation of Formulation 1

- prepare solution1a by dissolving BE1 in chloroform so that the concentration of BE1 is 10 mg/ml;
- prepare solution1b by dissolving PEO and IM2 in a ration of 20:1 in weight in cyclohexanone with a concentration of 20 mg/ml;
- Formulation 1 is then prepared by mixing solution1a and solution1b in a desired ratio so that the mass ratio of BE1:PEO:IM2 is 1:1:0.2.

### b) Preparation of Formulation 2

- prepare solution2a by dissolving YE1 in chloroform so that the concentration of YE1 is 10 mg/ml;
- prepare solution2b by dissolving PEO and IM1 in a ration of 1:0.12 in weight in cyclohexanone with a concentration of 20 mg/ml;
- Formulation 2 is then prepared by mixing solution2a and solution2b in a desired ratio so that the mass ratio of YE1:PEO:IM1 is 1:1:0.12.

### c) Preparation of Formulation 3

- RE1 + PMMA in mass ratio of 1:1 are dissolved in acetonitrile so that the concentration is 80 mg/ml.

### d) Preparation of Formulation 4

- prepare solution4a by dissolving RE2 in chloroform so that the concentration is 10 mg/ml;
- prepare solution4b by dissolving PEO and IM1 in a ration of 1:0.2 in weight in cyclohexanone with a concentration of 20 mg/ml;
- Formulation 4 is then prepared by mixing solution4a and solution4b in a desired ratio so that the mass ratio of RE2:PEO:IM1 is 1:1:0.2.

The Formulations can be used to prepare thin films, as required for the devices according to the present invention. The solid powder of the compositions can also be obtained by evaporating the solvents of the above prepared solutions.

### Example 3

### Substrate and layout for OLECs

The flexible poly(ethylene naphthalate) (PEN) is used as substrate for OLECs

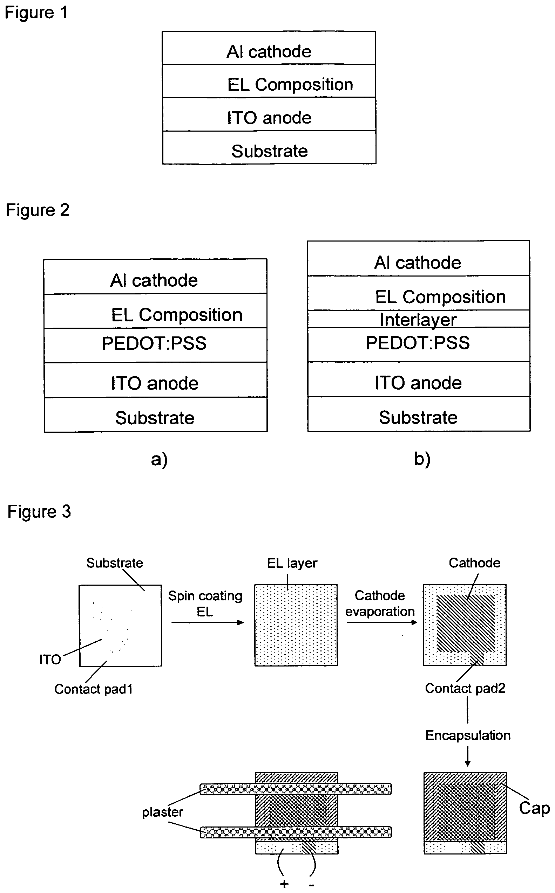

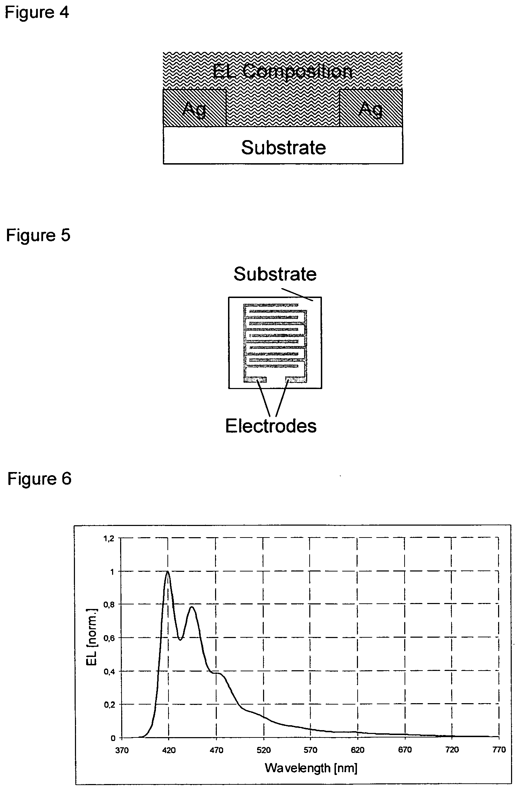

For OLECs with the sandwiched structure, as depicted in Figure 1 and Figure 2, 150 nm ITO is sputtered on PEN using a mask, as shown in Figure 3. It will be referred hereafter to as Sub1. Sub1 has a dimension of 3x3 cm, and a OLEC pixel of 2x2 cm.
For OLECs with planar interdigital electrode structure, as shown in Figure 4, 100 nm Ag is vacuum evaporated on PEN substrate using a shadow mask, as schematically shown in Figure 5. The interdigital electrodes have a finger width of 2 mm and finger distance of 200 µm. It will be referred hereafter to as Sub2.

### Example 4

### Blue OLECs using BE1

OLEC1 using BE1 in the emissive layer, in a sandwiched structure as shown in Figure 2a, is prepared according to the following steps:
1.) PDEOT (Baytron P Al 4083) is deposited with a thickness of 80 nm onto Sub1 by spin coating and then heated for 10 min. at 120°C;
2.) The emissive layer is deposited by spin-coating Formulation 1 yielding a layer with a thickness of 300 nm in the glove-box;
3.) The device is heated at 120°C for 30 min. to remove residual solvent;
4.) An Al (150 nm) cathode is deposited by evaporation onto the emissive layer;
5.) The device is encapsulated according to the method as described in Example 8.

OLEC2 using IL1 as interlayer and BE1 as emissive layer, in a sandwiched structure as shown in Figure 2b), is prepared according to the following steps:
1.) see step 1.) for the preparation of OLEC1;
2.) see step 2.) for the preparation of OLEC1;
3.) 20 nm IL1 is deposited by spin coating from a toluene solution having a concentration of 0.5 wt% in a glove-box;
4.) see step 3.) for the preparation of OLEC 1;
5.) see step 4.) for the preparation of OLEC 1;
6.) see step 5.) for the preparation of OLEC 1.

OLEC1 and OLEC2 are analyzed according to methods well known to one skilled person. Electroluminescent spectrums are recorded. At a voltage of 6 V, both OLECs shows bright blue electroluminescent emission. The electroluminescent spectrum of OLEC1 after applied 6 V for 5 min. is shown in Figure 6, showing a broad blue emission between 400 and 500 nm. OLEC2 has a similar electroluminescent spectrum as compared to OLEC1.The stability is tested under constant driving voltage. OLEC1 operates for 30 min.. The interlayer in OLEC2 significantly improves operating time of the device. OLEC2 operates for at least 6 hrs..

### Example 5

### Red OLECs using RE1

OLEC3 using RE1 in the emissive layer, in a sandwiched structure as shown in Figure 2a, is prepared in analogy to the preparation of OLEC1. However, in step 2 of the preparation procedure of OLEC3 Formulation 3 is used instead of Formulation 1.

OLEC4 using RE1 in the emissive layer, in a planar structure as shown in Figure 4, is prepared in the following steps:
1.) Emissive layer is deposited by spin-coating Formulation 3 on substrate Sub2 yielding a layer with a thickness of 200 to 300 nm in glove-box;
2.) The device is heated at 120°C for 30 min. to remove the residual solvent in glove-box;
3.) The device is encapsulated according to the method as described in Example 8.

The OLEC3 and OLEC4 are analyzed according to methods well known to one skilled person. At 4 V, OLEC3 shows bright red electroluminescent emission. In OLEC4, light emission comes from the area near to the negative electrodes fingers after applied 10 V after 2 min.. The stability is tested under constant driving voltage. OLEC3 works for more than 7hrs at 4 V. OLEC4 still works at 12V for at least one day.

### Example 6

### Red OLECs using RE2

OLEC5 using RE2 in the emissive layer, in a sandwiched structure as shown in Figure 2a, is prepared in analogy to the preparation of OLEC1.

However, in step 2 of the preparation procedure of OLEC5 Formulation 4 is used instead of Formulation 1.

OLEC6 using RE2 in the emissive layer, in a planar structure as shown in Figure 4, is prepared in analogy to the preparation of OLEC4. However, in step 1 of the preparation procedure of OLEC6 Formulation 4 is used instead of Formulation 3.

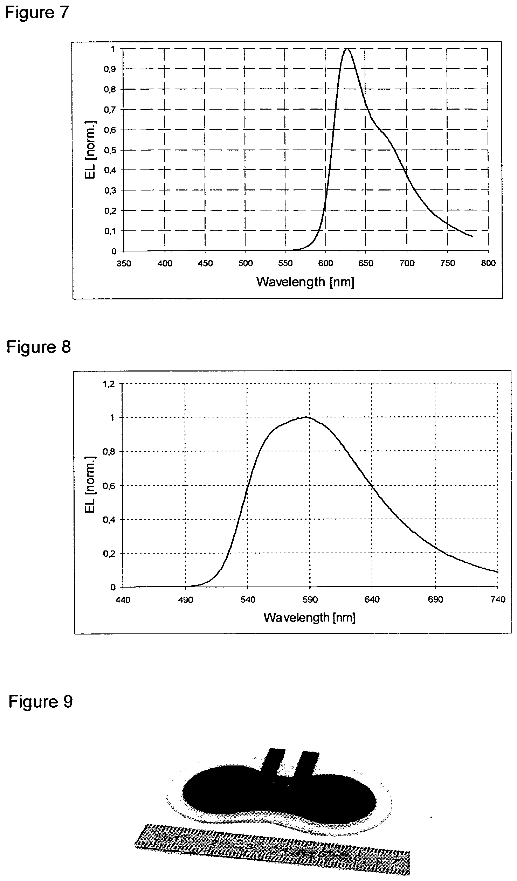

The OLEC5 and OLEC6 are investigated according to methods well known to one skilled person. Electroluminescent spectrums are recorded. At 3.5 V, OLEC5 shows bright red electroluminescent emission. Figure 7 depicts the electroluminescent spectrum of OLEC5 at 4 V after 5 min.. Figure 7 shows a broad red emission from 580 to 750 nm. In OLEC6, light emission comes from the area near to the negative electrodes fingers after having applied 9 V for 2 min.. The stability is investigated under constant driving voltage. OLEC5 works for more than 4 days at 4 V. OLEC6 works at 9 V for at least 4 days.

### Example 7

### Yellow OLECs using YE1

OLEC7 using YE1 in the emissive layer, in a sandwiched structure as shown in Figure 2a, is prepared in analogy to the preparation of OLEC1. However, in step 2 of the preparation procedure of OLEC7 Formulation 2 is used instead of Formulation 1.

OLEC8 using YE1 in the emissive layer, in a planar structure as shown in Figure 4, is prepared in analogy to the preparation of OLEC4. However, in step 1 of the preparation procedure of OLEC8 Formulation 2 is used instead of Formulation 3.

The OLEC7 and OLEC8 are investigated according to methods well known to one skilled person. Electroluminescent spectrums are recorded. At 3.5V, OLEC7 shows bright red electroluminescent emission. Figure 8 shows the electroluminescent spectrum of OLEC7 at 3.5 V after 5 min.. Figure 8 shows a broad blue emission from 500 to 700 nm with a maximal peak close to 590 nm. In OLEC8, light emission comes from the area near to the negative electrodes fingers at 8 V after 2 min.. The stability is investigated under constant driving voltage. OLEC7 works for at least 5 days at 4 V. OLEC8 works for at least 5 days at 8 V.

### Example 8

### Encapsulation

Encapsulation of OLEC1 to OLEC8 is achieved using a UV-cured resin, UV Resin T-470/UR7114 (Nagase Chemtex Corporation), and a PEN cap, which is smaller than the substrate to leave the contact pads free, as shown in step 4 of Figure 3. The UV-resin is applied at first on the edge of the pixel, and the cap is then located on top of them. Then the device is exposed to UV light for 30 seconds. All theses are done in glove-box.

### Example 9

### Device for therapeutic and/or cosmetic applications

The final devices for using in therapeutic and cosmetic applications can be realised, e.g., by attaching the OLECs devices to plasters, as shown in Figure 3. The external power source can be applied through the contact pads.

A battery is a preferred power source for the devices, particularly preferred is the printed thin film battery for light weight. The printed thin film battery can be acquired, for example from Fraunhofer Institute, as shown in Figure 9.

In some treatments, the device should be driven in pulse mode. Additionally, it is also reported that the stability of OLECs can be increased when operated in a pulse voltage scheme. Therefore a controller, particularly a pocket portable one, for pulse driving, is desired. This can be realised by using a commercially available flasher unit or blinker unit. Further such flasher unit can be integrated in the power unit, according to the principle of general trigger circuit, as for example shown in Fachkunde Elektrotechnik, Verlag Europa-Lehrmittel, Nourney, Vollmer GmbH & Co., 5657 Haan-Gruiten, 227.

### Example 10

### Treatment of crow's feet

OLEC7 comprising YE1 in the emissive layer can be used for the treatment and/or prophylaxis of wrinkles. The device is encapsulated according to the procedure in Example 8. A plaster is prepared according to Example 9 having a printed battery as power supply. The battery on each plaster supplies energy for a irradiation time of 30 min..

A 20-week pilot study with 18 female human subjects in the age between 30 and 40 years is conducted according to standard methods well known to the person skilled in the art. One of the main selection criteria for the inclusion within the study is the occurrence of crown's feet with almost equal manifestation on both sides of the face, i.e. in proximity to the left and right eye. Each subject is treated on the right hand side with a plaster comprising OLEC7 for 30 min. every second day for 20 weeks. Comparison of the skin in proximity of the left eye and right eye reveals a significant improvement of the skin on the treated side. The crow's feet are shorter and less deeper. The skin treated with light emitted by the OLEC device appears smoother as compared to the untreated skin.

### Example 11

### Treatment of acne vulgaris

Two plaster are prepared according to the procedures as outlined herein. The first plaster comprises OLEC1 emitting blue light, the second one comprises OLEC5 emitting red light. The plaster comprising the OLEC has squared shape with 9 cm², but any other customized shape is possible.
A 3-week pilot study with 26 subjects is conducted according to standard methods well known to the person skilled in the art. The subjects have Fitzpatrick skin types II to IV with mild to severe symmetric facial acne vulgaris. The right half of the forehead is treated with two different plasters in an alternating way. Overall 8 treatments are carried out. The first treatment is done by employing a first plaster emitting blue light (OLEC1) for 20 min.. Three days later the same skin is treated with a second plaster emitting red light (OLEC5) for 30 min. Further three days later the same skin is again treated with a first plaster, and so on. Comparing the left hand side and the right hand side of the foreheads of the treated subjects reveals a significant improvement of the treated skin as compared to the untreated skin. Redness of the skin is significantly reduced. Furthermore, mean lesion count reduction is also significant.

## Claims

1. Organic light emitting electrochemical cell (OLEC) comprising a composition comprising at least one ionic species and at least one organic electroluminescent compound for the use in medicine, **characterized in that** the device comprises a power supply which is a printable battery.

2. OLEC according to claim 1, **characterized in that** the at least one organic electroluminescent compound is selected from fluorescent emitter materials, phosphorescent emitter materials, and emissive organo metallic complexes.

3. OLEC according to claim 1 or 2, **characterized in that** the ionic species is cationic.

4. OLEC according to one or more of claims 1 to 3, **characterized in that** the ionic species is anionic.

5. OLEC according to one or more of claims 1 to 4 for use for the treatment and/or prophylaxis of skin diseases and/or skin-related conditions.

6. OLEC according to one or more of claims 1 to 5 for the use for the treatment and/or prophylaxis of skin diseases and/or skin-related conditions selected from acne, psoriasis, eczema, dermatitis, atopic dermatitis, edema, vitiligo, Bowens disease, tumors, pre-malignant tumors, malignant tumors, basal cell carcinomas, squamous cell carcinomas, secondary metastases, cutaneous T-cell lymphomas, solar keratosis, arsenical keratosis, radiodermatitis, and cellulite.

7. OLEC according to one or more of claims 1 to 6 for the use for the treatment and/or prophylaxis of infections and inflammatory, neurological, and psychological diseases and/or conditions.

8. OLEC according to one or more of claims 1 to 7 for the use in photodynamic therapy (PDT).

9. OLEC according to one or more of claims 1 to 8 for the use for the treatment and/or prophylaxis of jaundice and crigler naijar.

10. OLEC according to one or more of claims 1 to 9, **characterized in that** the device covers an area to be treated and emits electromagnetic radiation to cause said treatment and/or prophylaxis of the area, wherein the OLEC has an extent of at least 0.5 cm².

11. OLEC according to one or more of claims 1 to 10, **characterized in that** the device is an ambulatory device and comprises an attachment means for attaching the device to a patient.

12. OLEC according to one or more of claims 1 to 11, **characterized in that** the device is a plaster, bandage, blanket, sleeping bag, sleeve, implantable probe, nasogastric tube, chest drain, pad, stent, and patch.

## Patentansprüche

1. Organische lichtemittierende elektrochemische Zelle (organic light emitting electrochemical cell - OLEC) enthaltend eine Zusammensetzung enthaltend mindestens eine ionische Spezies und mindestens eine organische Elektrolumineszenzverbindung für die Verwendung in der Medizin, **dadurch gekennzeichnet, dass** die Vorrichtung eine Stromquelle enthält, bei der es sich um eine druckbare Batterie handelt.

2. OLEC nach Anspruch 1, **dadurch gekennzeichnet, dass** die mindestens eine organische Elektrolumineszenzverbindung aus fluoreszierenden Emittermaterialien, phosphoreszierenden Emittermaterialien und emittierenden metallorganischen Komplexen ausgewählt ist.

3. OLEC nach Anspruch 1 oder 2, **dadurch gekennzeichnet, dass** die ionische Spezies kationisch ist.

4. OLEC nach einem oder mehreren der Ansprüche 1 bis 3, **dadurch gekennzeichnet, dass** die ionische Spezies anionisch ist.

5. OLEC nach einem oder mehreren der Ansprüche 1 bis 4 zur Verwendung bei der Behandlung und/oder Prophylaxe von Hautkrankheiten und/oder mit der Haut in Zusammenhang stehenden Leiden.

6. OLEC nach einem oder mehreren der Ansprüche 1 bis 5 für die Verwendung bei der Behandlung und/oder Prophylaxe von Hautkrankheiten und/oder mit der Haut in Zusammenhang stehenden Leiden, die aus Akne, Psoriasis, Ekzem, Dermatitis, atopischer Dermatitis, Ödem, Vitiligo, Morbus Bowen, Tumoren, prämalignen Tumoren, malignen Tumoren, Basalzellkarzinomen, Plattenepithelkarzinomen, sekundären Metastasen, kutanen T-Zell-Lymphomen, solarer Keratose, Arsenkeratose, Radiodermatitis und Cellulitis ausgewählt sind.

7. OLEC nach einem oder mehreren der Ansprüche 1 bis 6 für die Verwendung bei der Behandlung und/oder Prophylaxe von Infektionen und Entzündungs-, neurologischen und psychologischen Krankheiten und/oder Leiden.

8. OLEC nach einem oder mehreren der Ansprüche 1 bis 7 für die Verwendung in der photodynamischen Therapie (PDT).

9. OLEC nach einem oder mehreren der Ansprüche 1 bis 8 für die Verwendung bei der Behandlung und/oder Prophylaxe von Gelbsucht und Crigler-Naijar.

10. OLEC nach einem oder mehreren der Ansprüche 1 bis 9, **dadurch gekennzeichnet, dass** die Vorrichtung eine zu behandelnde Fläche bedeckt und elektromagnetische Strahlung abgibt, um die genannte Behandlung und/oder Prophylaxe der Fläche herbeizuführen, wobei die OLEC eine Ausdehnung von mindestens 0,5 cm² aufweist.

11. OLEC nach einem oder mehreren der Ansprüche 1 bis 10, **dadurch gekennzeichnet, dass** es sich bei der Vorrichtung um eine bewegbare Vorrichtung handelt, die ein Befestigungsmittel aufweist, um die Vorrichtung an einem Patienten zu befestigen.

12. OLEC nach einem oder mehreren der Ansprüche 1 bis 11, **dadurch gekennzeichnet, dass** es sich bei der Vorrichtung um ein Pflaster, eine Bandage, eine Decke, einen Schlafsack, eine Hülle, eine implantierbare Sonde, eine nasogastrale Sonde, eine Thoraxdrainage, eine Vorlage, einen Stent und einen Patch handelt.

## Revendications

1. Cellule électrochimique à émission de lumière organique (OLEC) comprenant une composition comprenant au moins une espèce ionique et au moins un composé électroluminescent organique pour une utilisation en médecine, **caractérisée en ce que** le dispositif comprend une alimentation qui est un accumulateur imprimable.

2. OLEC selon la revendication 1, **caractérisée en ce que** l'au moins un composé électroluminescent organique est choisi parmi des matériaux d'émetteur fluorescents, des matériaux d'émetteur phosphorescents et des complexes organométalliques émissifs.

3. OLEC selon la revendication 1 ou 2, **caractérisée en ce que** l'espèce ionique est cationique.

4. OLEC selon une ou plusieurs des revendications 1 à 3, **caractérisée en ce que** l'espèce ionique est anionique.

5. OLEC selon une ou plusieurs des revendications 1 à 4 pour une utilisation pour le traitement et/ou la prophylaxie de maladies de la peau et/ou de conditions concernant la peau.

6. OLEC selon une ou plusieurs des revendications 1 à 5 pour une utilisation pour le traitement et/ou la prophylaxie de maladies de la peau et/ou de conditions concernant la peau choisies parmi l'acné, le psoriasis, l'eczéma, la dermatite, la dermatite atopique, l'oedème, le vitiligo, la maladie de Bowens, les tumeurs, les tumeurs prémalignes, les tumeurs malignes, les carcinomes basocellulaires, les carcinomes à cellules squameuses, les métastases secondaires, les lymphomes à lymphocytes T cutanés, la kératose solaire, la kératose arsénicale, la radiodermatite et la cellulite.

7. OLEC selon une ou plusieurs des revendications 1 à 6 pour une utilisation pour le traitement et/ou la prophylaxie d'infections et de maladies et/ou de conditions inflammatoires, neurologiques et psychologiques.

8. OLEC selon une ou plusieurs des revendications 1 à 7 pour une utilisation au niveau de la thérapie photodynamique (PDT).

9. OLEC selon une ou plusieurs des revendications 1 à 8 pour une utilisation pour le traitement et/ou la prophylaxie de la jaunisse et du syndrome de Crigler-Naijar.

10. OLEC selon une ou plusieurs des revendications 1 à 9, **caractérisée en ce que** le dispositif couvre une zone à traiter et émet un rayonnement électromagnétique pour mettre en oeuvre ledit traitement et/ou ladite prophylaxie de la zone, dans laquelle l'OLEC présente une étendue d'au moins 0,5 cm².

11. OLEC selon une ou plusieurs des revendications 1 à 10, **caractérisée en ce que** le dispositif est un dispositif ambulatoire et comprend un moyen de fixation pour fixer le dispositif sur un patient.

12. OLEC selon une ou plusieurs des revendications 1 à 11, **caractérisée en ce que** le dispositif est un pansement, un bandage, une couverture, un sac de couchage, une chemise, une sonde implantable, un tube nasogastrique, un drain thoracique, une compresse, un stent et un patch.
